# EUROPEAN PATENT APPLICATION

(11) **EP 4 756 025 A1**
(43) Date of publication of application: **10.06.2026**
(21) Application number: 24848344.8
(22) Date of filing: 01.08.2024
(51) Int. Cl.: C12N 15/113

(54) **DSRNA TARGETING MAPT AND PHARMACEUTICAL USE THEREOF**

(30) Priority: 01.08.2023 CN 202310956218; 23.01.2024 CN 202410092507
(71) Applicant: Tuojie Biotech (Shanghai) Co., Ltd., Shanghai 201203 (CN)
(72) Inventor: WEN, Yuhao, Shanghai 201203 (CN); CHEN, Di, Shanghai 201203 (CN); LIN, Xiaoyan, Shanghai 201203 (CN); LI, Yunfei, Shanghai 201203 (CN)
(74) Representative: Regimbeau
(86) International application number: PCT/CN2024/109181
(87) International publication number: WO 2025/026390

(57) **Abstract**

A dsRNA targeting MAPT, a dsRNA conjugate, and a pharmaceutical use. A pharmaceutical composition, cell or kit comprising the dsRNA, and a method of using the dsRNA for treating and/or preventing related disorders suffered by a subject.

## Description

### TECHNICAL FIELD

The present disclosure pertains to the field of biopharmaceuticals and particularly relates to a dsRNA targeting the microtubule-associated protein tau gene (MAPT), a composition, and pharmaceutical use thereof.

### BACKGROUND

The microtubule-associated protein tau gene (MAPT), located in the chromosome 17q21.31 region, encodes the microtubule-associated protein tau. The tau protein is a microtubule-associated protein. Under normal physiological conditions, it is primarily located in the axons of adult neurons and regulates the stability of tubulin assembly. Exons 9, 10, 11, and 12 of the MAPT gene encode the microtubule-binding repeats of the tau protein. Due to selective splicing of exon 10, subtypes of the tau protein are classified as 3R-tau and 4R-tau, where "R" refers to the number of repeats in the microtubule-binding domain. In the adult human brain, the ratio of the 3R-tau protein subtype to the 4R-tau protein subtype is approximately 1. The 3R-tau/4R-tau ratio is imbalanced in disease states. Deviating from the normal 3R/4R tau ratio is a feature of neurodegenerative tauopathies.

The tau protein is very high in proline residue content and lysine residue content and has more than 80 potential phosphorylation sites, which makes tau prone to hyperphosphorylation-induced cross-linking and aggregation via condensation with other tau molecules under pathological conditions. The phosphorylation level of tau under pathological conditions is 2-3 times the normal level. This hyperphosphorylation causes the tau protein to detach from microtubules, resulting in elevated cytoplasmic tau levels. Tau molecules aggregate to form poorly soluble fiber bundles, eventually leading to neurofibrillary tangles-an important pathological feature of Alzheimer's disease (AD). Tau pathology is also observed in other neurodegenerative dementias, such as frontotemporal dementia and parkinsonism linked to chromosome 17 (FTDP-17), Pick's disease (PiD), progressive supranuclear palsy (PSP), corticobasal degeneration (CBD), argyrophilic grain disease (AGD), tangle-only dementia (TOD), and chronic traumatic encephalopathy (CTE). In particular, patients with FTDP-17 carry many exon and intron mutations in the tau gene, resulting in tau accumulation. These findings suggest that abnormalities in the tau protein lead to accumulation of the tau protein and degeneration of neurons.

Currently, there is a lack of acceptable methods for treating such neurodegenerative diseases. Therefore, there is a need for drugs that selectively and effectively inhibit or regulate MAPT gene expression, in order to effectively treat subjects with MAPT-related diseases, such as Alzheimer's disease, frontotemporal dementia (FTD), PSP, or other tauopathies. The objective of the present disclosure is to provide a dsRNA and a method for treating such diseases.

### SUMMARY

The present disclosure provides a dsRNA targeting MAPT.

In some embodiments, the present disclosure provides a dsRNA comprising a sense strand and an antisense strand that form a double-stranded region, wherein:
the sense strand comprises a sequence of at least 15 (e.g., 16, 17, 18, 19, 20, or 21) contiguous nucleotides differing from any one of the nucleotide sequences of SEQ ID NO: 1 to SEQ ID NO: 10 and SEQ ID NO: 21 to SEQ ID NO: 78 by no more than 3 (e.g., 0, 1, 2, or 3) nucleotides;
the antisense strand comprises a sequence of at least 15 (e.g., 16, 17, 18, 19, 20, or 21) contiguous nucleotides differing from any one of the nucleotide sequences of SEQ ID NO: 11 to SEQ ID NO: 20 and SEQ ID NO: 79 to SEQ ID NO: 136 by no more than 3 (e.g., 0, 1, 2, or 3) nucleotides.

In some embodiments, the sense strand comprises a sequence of at least 17 contiguous nucleotides differing from any one of the nucleotide sequences of SEQ ID NO: 1 to SEQ ID NO: 10 and SEQ ID NO: 21 to SEQ ID NO: 78 by no more than 3 nucleotides, and the antisense strand comprises a sequence of at least 17 contiguous nucleotides differing from any one of the nucleotide sequences of SEQ ID NO: 11 to SEQ ID NO: 20 and SEQ ID NO: 79 to SEQ ID NO: 136 by no more than 3 nucleotides.

In some embodiments, the sense strand comprises a sequence of at least 19 contiguous nucleotides differing from any one of the nucleotide sequences of SEQ ID NO: 1 to SEQ ID NO: 10 and SEQ ID NO: 21 to SEQ ID NO: 78 by no more than 3 nucleotides, and/or the antisense strand comprises a sequence of at least 21 contiguous nucleotides differing from any one of the nucleotide sequences of SEQ ID NO: 11 to SEQ ID NO: 20 and SEQ ID NO: 79 to SEQ ID NO: 136 by no more than 3 nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the dsRNA of the present disclosure comprises one or two blunt ends. In some specific embodiments, each strand of the dsRNA independently comprises an overhang comprising 1 to 2 unpaired nucleotides. In some embodiments, the dsRNA of the present disclosure comprises an overhang at the 3' end of the antisense strand of the dsRNA.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. The length ratio of the sense strand to the antisense strand of the dsRNA provided by the present disclosure may be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand of the dsRNA is 19/21, 21/23, or 23/25. In some embodiments, the length ratio of the sense strand to the antisense strand of the dsRNA is 19/21.

In some embodiments, the sense strand comprises or is selected from the group consisting of the nucleotide sequence set forth in any one of the following: SEQ ID NO: 1 to SEQ ID NO: 10 and SEQ ID NO: 21 to SEQ ID NO: 78.

In some embodiments, the antisense strand comprises or is selected from the group consisting of the nucleotide sequence set forth in any one of the following: SEQ ID NO: 11 to SEQ ID NO: 20 and SEQ ID NO: 79 to SEQ ID NO: 136.

In some embodiments, the dsRNA of the present disclosure comprises or is selected from the group consisting of any one of the following groups:
group 1): a sense strand set forth in SEQ ID NO: 1 and an antisense strand set forth in SEQ ID NO: 11;
group 2): a sense strand set forth in SEQ ID NO: 2 and an antisense strand set forth in SEQ ID NO: 12;
group 3): a sense strand set forth in SEQ ID NO: 3 and an antisense strand set forth in SEQ ID NO: 13;
group 4): a sense strand set forth in SEQ ID NO: 4 and an antisense strand set forth in SEQ ID NO: 14;
group 5): a sense strand set forth in SEQ ID NO: 5 and an antisense strand set forth in SEQ ID NO: 15;
group 6): a sense strand set forth in SEQ ID NO: 6 and an antisense strand set forth in SEQ ID NO: 16;
group 7): a sense strand set forth in SEQ ID NO: 7 and an antisense strand set forth in SEQ ID NO: 17;
group 8): a sense strand set forth in SEQ ID NO: 8 and an antisense strand set forth in SEQ ID NO: 18;
group 9): a sense strand set forth in SEQ ID NO: 9 and an antisense strand set forth in SEQ ID NO: 19; and
group 10): a sense strand set forth in SEQ ID NO: 10 and an antisense strand set forth in SEQ ID NO: 20.

In some embodiments, at least one nucleotide in the sense strand and/or the antisense strand is a modified nucleotide. In some embodiments, all nucleotides in the sense strand and/or the antisense strand are modified nucleotides. In some embodiments, all nucleotides are modified nucleotides.

In some embodiments, the modified nucleotides are selected from the group consisting of a 2'-fluoro-modified nucleotide, a 2-methoxy-modified nucleotide, and a nucleotide in which the 5' end of the nucleoside is modified by a trans-vinylphosphodiester group.

In some embodiments, three contiguous nucleotides in the sense strand of the dsRNA are 2'-fluoro-modified nucleotides. In some embodiments, in the sense strand of the dsRNA, in the direction from the 5' end to the 3' end, the three contiguous nucleotides at positions 7-9 of the sense strand are 2'-fluoro-modified nucleotides. In some embodiments, the three contiguous nucleotides at positions 7-9 of the sense strand of the dsRNA are 2'-fluoro-modified nucleotides, and the nucleotides at the remaining positions of the sense strand are all 2'-methoxy-modified nucleotides.

In some embodiments, in the direction from the 5' end to the 3' end, each of the nucleotides at positions 2, 6, 12, 14, and 16 of the antisense strand is independently a 2'-fluoro-modified nucleotide. In some embodiments, in the direction from the 5' end to the 3' end, the nucleotide at position 2, 4, 6, 10, 12, 14, 16, or 18 of the antisense strand is independently a 2'-fluoro-modified nucleotide. In some embodiments, the nucleotides at the remaining positions of the antisense strand are all 2'-methoxy-modified nucleotides. In some embodiments, the 5' end of the antisense strand comprises one phosphorus-containing group, and the phosphorus-containing group is a 5'-vinylphosphodiester group (5'-VP). When the 5'-end phosphorus-containing group is a 5'-vinylphosphodiester group (5'-Vp), the 5'-Vp may be a 5'-*E*-VP isomer (i.e., a trans-vinylphosphodiester group), a 5'-Z-VP isomer (i.e., a cis-vinylphosphodiester group), or a mixture thereof. In some embodiments, the 5' end of the antisense strand comprises one phosphorus-containing group, and the phosphorus-containing group is a 5'-*E*-VP isomer (i.e., a trans-vinylphosphodiester group).

In some embodiments, at least one phosphodiester group in the sense strand and/or the antisense strand is a phosphodiester group with a modification group. The modification group causes the dsRNA to have increased stability in a biological sample or environment. In some embodiments, the phosphodiester group with a modification group is a phosphorothioate diester group. In some embodiments, the phosphodiester group with a modification group is a 5'-vinylphosphodiester group.

In some embodiments, the phosphodiester group with a modification group is present in at least one position selected from the group consisting of the following positions:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

In some embodiments, the sense strand and/or the antisense strand comprise(s) multiple phosphodiester groups with a modification group, and the phosphodiester groups with a modification group are present in multiple positions described above.

In some embodiments, the phosphodiester groups with a modification group are present:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

In some embodiments, in the direction from the 5' end to the 3' end, at least one of the nucleotides at positions 2 to 8 (e.g., position 2, position 3, position 4, position 5, position 6, position 7, and position 8) of the antisense strand comprises a chemical modification represented by formula (I) or a pharmaceutically acceptable salt thereof, and the chemical modification represented by formula (I) is selected from the group consisting of: 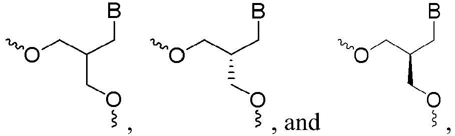 wherein B represents the bases at positions corresponding to positions 2-8 of the 5' end of the antisense strand.

In some embodiments, the sense strand is selected from the group consisting of or comprises the nucleotide sequence set forth in any one of SEQ ID NO: 137 to SEQ ID NO: 201.

In some embodiments, the antisense strand is selected from the group consisting of or comprises the nucleotide sequence set forth in any one of SEQ ID NO: 202 to SEQ ID NO: 266.

In some more specific embodiments, the dsRNA comprises or is selected from the group consisting of any one of the following combinations of a sense strand and an antisense sequence:
a sense strand set forth in SEQ ID NO: 137 and an antisense strand set forth in SEQ ID NO: 202;
a sense strand set forth in SEQ ID NO: 138 and an antisense strand set forth in SEQ ID NO: 203;
a sense strand set forth in SEQ ID NO: 139 and an antisense strand set forth in SEQ ID NO: 204;
a sense strand set forth in SEQ ID NO: 140 and an antisense strand set forth in SEQ ID NO: 205;
a sense strand set forth in SEQ ID NO: 141 and an antisense strand set forth in SEQ ID NO: 206;
a sense strand set forth in SEQ ID NO: 142 and an antisense strand set forth in SEQ ID NO: 207;
a sense strand set forth in SEQ ID NO: 143 and an antisense strand set forth in SEQ ID NO: 208;
a sense strand set forth in SEQ ID NO: 144 and an antisense strand set forth in SEQ ID NO: 209;
a sense strand set forth in SEQ ID NO: 145 and an antisense strand set forth in SEQ ID NO: 210; and
a sense strand set forth in SEQ ID NO: 146 and an antisense strand set forth in SEQ ID NO: 211.

In some embodiments, the dsRNA is linked to one or more lipophilic groups. In some embodiments, the sense strand of the dsRNA is linked to one or more lipophilic groups. In some embodiments, the one or more lipophilic groups are linked to one or more nucleotides in the sense strand of the dsRNA; in some embodiments, the one or more lipophilic groups are linked to one or more nucleotides in the antisense strand of the dsRNA; in some embodiments, the one or more lipophilic groups are linked to multiple nucleotides in the sense strand and the antisense strand of the dsRNA.

In some embodiments, the one or more lipophilic groups are linked to one or more bases; in some embodiments, the one or more lipophilic groups are linked to one or more sugar rings; in some embodiments, the one or more lipophilic groups are linked to one or more internucleoside linking groups.

In some embodiments, the one or more lipophilic groups are linked to at least one of the following positions:
the 1st nucleotide of the 5' end or 3' end of the sense strand; the 1st nucleotide of the 5' end or 3' end of the antisense strand; the 1st nucleotide of the 3' end of the antisense strand;
the nucleotide at a position in the middle of the sense strand; and the nucleotide at a position in the middle of the antisense strand.

In some embodiments, in the direction from the 5' end to the 3' end, the one or more lipophilic groups are linked to the 2nd-8th nucleotides of the sense strand; in some embodiments, the one or more lipophilic groups are linked to the 2nd-8th nucleotides of the antisense strand. In some embodiments, the lipophilic group is linked to the 6th nucleotide of the sense strand.

In some embodiments, the lipophilic group comprises a saturated or unsaturated C₄-₃₀ hydrocarbon chain, and optionally a functional group selected from the group consisting of halogen, alkoxy, hydroxy, amine, carboxylic acid, sulfonate, phosphate, thiol, azide, and alkyne.

In some embodiments, the lipophilic group comprises a saturated or unsaturated C₆₋₁₈ hydrocarbon chain; in some embodiments, the lipophilic group comprises a saturated or unsaturated C₁₆ hydrocarbon chain.

In some embodiments, the saturated or unsaturated C₁₆ hydrocarbon chain is linked to the 1st nucleotide of the 5' end of the sense strand.

In some embodiments, the sense strand comprises or is selected from the group consisting of the sequence set forth in any one of SEQ ID NO: 267 to SEQ ID NO: 276, and/or the antisense strand comprises or is selected from the group consisting of the sequence set forth in any one of SEQ ID NO: 277 to SEQ ID NO: 286.

In some embodiments, the dsRNA comprises or is selected from the group consisting of any one of the following groups:
a sense strand set forth in SEQ ID NO: 267 and an antisense strand set forth in SEQ ID NO: 277;
a sense strand set forth in SEQ ID NO: 268 and an antisense strand set forth in SEQ ID NO: 278;
a sense strand set forth in SEQ ID NO: 269 and an antisense strand set forth in SEQ ID NO: 279;
a sense strand set forth in SEQ ID NO: 270 and an antisense strand set forth in SEQ ID NO: 280;
a sense strand set forth in SEQ ID NO: 271 and an antisense strand set forth in SEQ ID NO: 281;
a sense strand set forth in SEQ ID NO: 272 and an antisense strand set forth in SEQ ID NO: 282;
a sense strand set forth in SEQ ID NO: 273 and an antisense strand set forth in SEQ ID NO: 283;
a sense strand set forth in SEQ ID NO: 274 and an antisense strand set forth in SEQ ID NO: 284;
a sense strand set forth in SEQ ID NO: 275 and an antisense strand set forth in SEQ ID NO: 285; and
a sense strand set forth in SEQ ID NO: 276 and an antisense strand set forth in SEQ ID NO: 286.

The present disclosure further provides a dsRNA comprising any one of the dsRNAs described above and a targeting ligand linked to the dsRNA. In some embodiments, the dsRNA and the targeting ligand are linked covalently or non-covalently.

In some embodiments, the targeting ligand targets the liver; in some embodiments, the targeting ligand binds to asialoglycoprotein receptor (ASGPR); in some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, and N-isobutyrylgalactosamine.

In some embodiments, the targeting ligand is linked to the 3' end of the sense strand of the dsRNA.

In some embodiments, the targeting ligand is linked to an end of the dsRNA by a phosphodiester group, a phosphorothioate diester group, or a phosphonic acid group; in some embodiments, the targeting ligand is linked to an end of the dsRNA by a phosphodiester group.

In some embodiments, the targeting ligand is indirectly linked to an end of the dsRNA by a phosphodiester group, a phosphorothioate diester group, or a phosphonic acid group; in some embodiments, the targeting ligand is indirectly linked to an end of the dsRNA by a phosphodiester group.

In some embodiments, the targeting ligand is directly linked to an end of the dsRNA by a phosphodiester group, a phosphorothioate diester group, or a phosphonic acid group; in some embodiments, the targeting ligand is directly linked to an end of the dsRNA by a phosphodiester group.

In some embodiments, the targeting ligand is directly linked to the 3' end of the sense strand of the dsRNA by a phosphodiester group or a phosphorothioate diester group; in some embodiments, the targeting ligand is directly linked to the 3' end of the sense strand of the dsRNA by a phosphodiester group.

In some embodiments, the targeting moiety of the targeting ligand consists of one or more targeting groups or targeting moieties, and the targeting ligand assists in directing the delivery of a therapeutic agent linked thereto to the desired target location. In some cases, the targeting moiety may bind to a cell or cellular receptor and initiate endocytosis to promote entry of the therapeutic agent into the cell. The targeting moiety may comprise a compound with affinity for a cellular receptor or a cell surface molecule or an antibody. Various targeting ligands comprising targeting moieties may be linked to therapeutic agents and other compounds to target the agents at cells and specific cellular receptors. In some embodiments, the types of the targeting moieties include carbohydrates, cholesterol and cholesterol groups, or steroids. Targeting moieties capable of binding to cellular receptors include saccharides such as galactose, galactose derivatives (e.g., N-acetyl-galactosamine, N-trifluoroacetylgalactosamine, N-propionylgalactosamine, N-n-butyrylgalactosamine, and N-isobutyrylgalactosamine), mannose, and mannose derivatives).

Targeting moieties that bind to asialoglycoprotein receptors (ASGPRs) are known to be particularly used for directing the delivery of oligomeric compounds to the liver. Asialoglycoprotein receptors are extensively expressed on liver cells (hepatocytes). Cellular receptor targeting moieties targeting ASGPRs include galactose and galactose derivatives. Specifically, clusters of galactose derivatives, including clusters consisting of 2, 3, 4, or more than 4 N-acetyl-galactosamine (GalNAc or NAG) molecules, can promote the uptake of certain compounds in hepatocytes. The GalNAc cluster coupled to the oligomeric compound is used for directing the composition to the liver, where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand may comprise 2, 3, 4, or more than 4 targeting moieties. In some embodiments, the targeting ligand disclosed herein may comprise 1, 2, 3, 4, or more than 4 targeting moieties linked to a branching group by L₂.

In some embodiments, each targeting moiety comprises a galactosamine derivative that is N-acetyl-galactosamine. Other sugars that may be used as targeting moieties and have affinity for asialoglycoprotein receptors may be selected from the group consisting of galactose, galactosamine, N-formyl-galactosamine, N-acetyl-galactosamine, N-propionyl-galactosamine, N-n-butyryl-galactosamine, N-isobutyryl-galactosamine, etc. In some embodiments, the targeting ligand in the present disclosure comprises N-acetylgalactosamine as a targeting moiety,

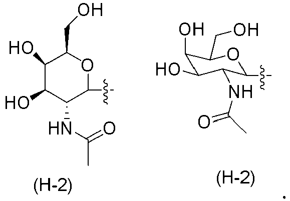

In some embodiments, the targeting ligand comprises three terminal galactosamines or galactosamine derivatives (such as N-acetyl-galactosamine), each of which has an affinity for asialoglycoprotein receptors. In some embodiments, the targeting ligand comprises three terminal N-acetyl-galactosamines (GalNAc or NAG) as targeting moieties.

In some embodiments, the targeting ligand comprises four terminal galactosamines or galactosamine derivatives (such as N-acetyl-galactosamine), each of which has an affinity for asialoglycoprotein receptors. In some embodiments, the targeting ligand comprises four terminal N-acetyl-galactosamines (GalNAc or NAG) as targeting moieties.

Terms commonly used in the art when referring to three terminal N-acetyl-galactosamines include tri-antennary, tri-valent, and trimer. Terms commonly used in the art when referring to four terminal N-acetyl-galactosamines include tetra-antennary, tetra-valent, and tetramer.

In some embodiments, the targeting ligand provided by the present disclosure is a compound as shown below or a pharmaceutically acceptable salt thereof: 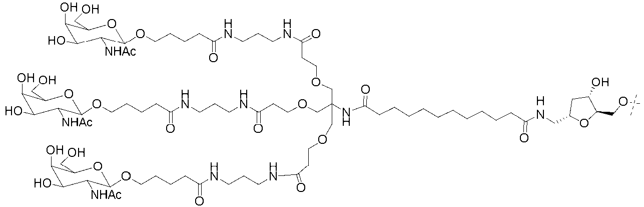 or

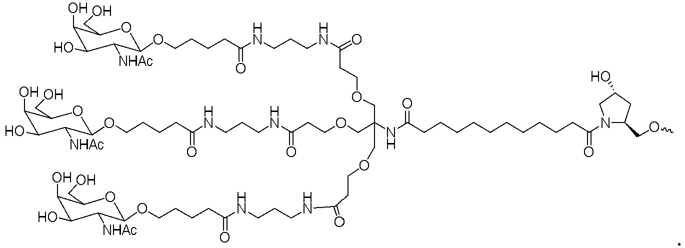

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In another aspect, the present disclosure provides a composition comprising the dsRNA described in the present disclosure, and one or more pharmaceutically acceptable excipients, such as vehicles, carriers, diluents, and/or delivery polymers. The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. Various delivery systems are known and can be used for the dsRNA of the present disclosure, e.g., encapsulation in liposomes, microparticles, microcapsules, recombinant cells capable of expressing the dsRNA, receptor-mediated endocytosis, and construction of a nucleic acid as part of a retroviral vector or other vectors.

"Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

In some embodiments, the pharmaceutical composition may further comprise a pharmaceutically acceptable auxiliary material and/or adjuvant, and the auxiliary material may be one or more of the various formulations or compounds conventionally used in the art. For example, the pharmaceutically acceptable auxiliary material may include at least one of a pH buffer, a protective agent, and an osmotic pressure regulator.

In some embodiments, a unit dose of the pharmaceutical composition is 0.001 mg-1000 mg.

In certain embodiments, the pharmaceutical composition comprises 0.01-99.99% of the aforementioned compound or a pharmaceutically acceptable salt thereof or an isotopically substituted form thereof based on the total weight of the composition. In certain embodiments, the pharmaceutical composition comprises 0.1-99.9% of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof. The pharmaceutical composition may be conveniently presented in a unit dosage form. In general, the pharmaceutical composition may be formulated into any suitable dosage form, such as, but not limited to, injections, tablets, capsules, gels, etc. The pharmaceutical composition includes, but is not limited to, a solution, an emulsion, and a liposome-containing formulation.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure provides use of the dsRNA or the composition of the present disclosure in the manufacture of a medicament for treating a disease in a subject; in some embodiments, the disease is selected from the group consisting of hepatic diseases; in some embodiments, the disease is selected from the group consisting of central neurodegenerative diseases. In some embodiments, the disease is a disease associated with MAPT gene expression. The present disclosure provides use of the dsRNA and/or the pharmaceutical composition of the present disclosure in the manufacture of a medicament for treating and/or preventing a disease associated with MAPT gene expression; in some embodiments, the disease is selected from the group consisting of neurodegenerative diseases associated with the MAPT gene; in some embodiments, the disease is selected from the group consisting of tauopathy, Alzheimer's disease (AD), frontotemporal dementia and parkinsonism linked to chromosome 17 (FTDP-17), frontotemporal dementia (FTD), progressive supranuclear palsy (PSP), corticobasal degeneration (CBD), chronic traumatic encephalopathy (CTE), epilepsy, Dravet syndrome (DS), Pick's disease (PiD), argyrophilic grain disease (AGD), and globular glial tauopathy (GGT).

The present disclosure provides use of the aforementioned dsRNA and/or pharmaceutical composition in the manufacture of a medicament for inhibiting MAPT expression.

In another aspect, the present disclosure provides a method for treating and/or preventing a disease in a subject, comprising administering to the subject the dsRNA and/or the composition of the present disclosure. In some embodiments, the disease is a disease associated with MAPT gene expression. In some embodiments, the disease is selected from the group consisting of tauopathy, Alzheimer's disease (AD), frontotemporal dementia and parkinsonism linked to chromosome 17 (FTDP-17), frontotemporal dementia (FTD), progressive supranuclear palsy (PSP), corticobasal degeneration (CBD), chronic traumatic encephalopathy (CTE), epilepsy, Dravet syndrome (DS), Pick's disease (PiD), argyrophilic grain disease (AGD), and globular glial tauopathy (GGT).

In another aspect, the present disclosure provides a method for reducing MAPT gene expression, comprising administering to a subject an effective amount or effective dose of the dsRNA and/or the pharmaceutical composition of the present disclosure.

In another aspect, the present disclosure provides a method for inhibiting MAPT mRNA expression *in vivo* in a subject, comprising administering to the subject the dsRNA and/or the pharmaceutical composition of the present disclosure.

In another aspect, the present disclosure provides a method for delivering an expression-inhibiting oligomeric compound to the liver *in vivo,* comprising administering to a subject the dsRNA and/or the pharmaceutical composition of the present disclosure.

In another aspect, the present disclosure provides a method for delivering a dsRNA inhibiting MAPT expression and/or replication *in vivo,* comprising administering to a subject the dsRNA and/or the pharmaceutical composition of the present disclosure.

In another aspect, the present disclosure provides a method for delivering an expression-inhibiting oligomeric compound to a central system *in vivo,* comprising administering to a subject the dsRNA or the composition described above.

In another aspect, the present disclosure provides a kit comprising the dsRNA and/or the pharmaceutical composition of the present disclosure.

In another aspect, the present disclosure provides a cell comprising the dsRNA of the present disclosure.

The dsRNA or the composition and the methods disclosed herein can reduce the level of a target mRNA in a cell, a cell population, a cell population, a tissue, or a subject, comprising administering to the subject a therapeutically effective amount of the dsRNA or the composition described in the present disclosure. The dsRNA is linked to a targeting ligand, thereby inhibiting the expression of the target mRNA in the subject. In some embodiments, the subject has been previously identified as having pathological up-regulation of the target gene in the targeted cell or tissue. The subject described in the present disclosure refers to a subject having (or suspected to have or susceptible to) a disease or disorder that would benefit from reduction or inhibition of target mRNA expression.

The delivery may be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In an optional embodiment, the pharmaceutical composition provided by the present disclosure may be administered by injection, e.g., intravenous, intramuscular, intradermal, subcutaneous, intraduodenal, or intraperitoneal injection.

In an optional embodiment, after the lipophilic group and/or the targeting ligand described above are/is linked to the dsRNA to form a conjugate, the conjugate can be packaged in a kit.

In some embodiments, when the dsRNA or the pharmaceutical composition described above is in contact with a target gene-expressing cell, the dsRNA or the pharmaceutical composition described above inhibits the expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry. In some embodiments, when the dsRNA or the pharmaceutical composition described above is in contact with a target gene-expressing cell, the dsRNA or the pharmaceutical composition described above results in a remaining expression percentage of the target gene mRNA of no greater than 99%, no greater than 95%, no greater than 90%, no greater than 85%, no greater than 80%, no greater than 75%, no greater than 70%, no greater than 65%, no greater than 60%, no greater than 55%, no greater than 50%, no greater than 45%, no greater than 40%, no greater than 35%, no greater than 30%, no greater than 25%, no greater than 20%, no greater than 15%, or no greater than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA or the pharmaceutical composition described above is in contact with a target gene-expressing cell, the dsRNA, while retaining on-target activity, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA or the pharmaceutical composition described above is in contact with a target gene-expressing cell, the dsRNA, while reducing on-target activity by at most 20%, at most 19%, at most 15%, at most 10%, at most 5%, or more than 1%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the dsRNA or the pharmaceutical composition described above is in contact with a target gene-expressing cell, the dsRNA, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry. The present disclosure further provides a method for preparing the dsRNA and/or the pharmaceutical composition of the present disclosure, comprising synthesizing the dsRNA and/or the pharmaceutical composition described in the present disclosure.

The present disclosure further provides a dsRNA, wherein one or more bases U, e.g., 1, 2, 3, 3, 5, 6, 7, 8, 9, 10, 11, or 12 bases U, of any dsRNA of the present disclosure are replaced with bases T. In some embodiments, all bases U are replaced with bases T. The pharmaceutically acceptable salts of the compounds described in the present disclosure are selected from the group consisting of inorganic salts and organic salts. The compounds described in the present disclosure can react with acidic or basic substances to form corresponding salts.

In another aspect, where the configuration is not specified, the compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof fall within the scope of the present disclosure.

In addition, when the configuration is not specified, the compounds and intermediates of the present disclosure can also be present in different tautomeric forms, and all such forms fall within the scope of the present disclosure. The term "tautomer" or "tautomeric form" refers to structural isomers of different energies that can interconvert via a low-energy barrier.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compounds of formula I and formula II. Each available hydrogen atom linked to a carbon atom may be independently replaced with a deuterium atom. Those skilled in the art can synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

Where the configurations are not specified, in the chemical structures of the compounds of the present disclosure, the bond " 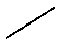 " indicates an unspecified configuration; that is, if chiral isomers exist in the chemical structures, the bond " 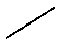 " may be " 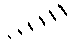 " or " 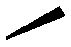 ", or includes both the configurations " 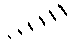 " and " 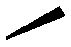 " simultaneously. Although all of the above structural formulas are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, the bond " 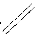 " does not specify a configuration; that is, the configuration of the bond " 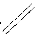 " may be an E configuration or a Z configuration, or includes both the E configuration and the Z configuration simultaneously.

WO2022028462A1 and WO2023274395A1 are incorporated in the present disclosure by reference in their entirety.

### Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless otherwise specified, in the context of the present disclosure, the terms "microtubule-associated protein tau gene", "MAPT", and "microtubule associated protein tau" are used interchangeably in the present disclosure. MAPT includes, but is not limited to, human MAPT, cynomolgus monkey MAPT, mouse MAPT, and rat MAPT, and their amino acids, complete coding sequences, and mRNA sequences are readily accessible using publicly available databases, e.g., GenBank, UniProt, OMIM, and the Macaca genome project website.

The term "MAPT" also refers to naturally occurring DNA sequence variations of the MAPT gene, such as a single nucleotide polymorphism (SNP) in the MAPT gene. Exemplary SNPs may be found in the dbSNP database.

The term "target sequence" refers to a contiguous portion of the nucleotide sequence of an mRNA molecule formed during the transcription of MAPT, including mRNA that is a product of RNA processing of a primary transcription product. The targeted portion in the target sequence should be long enough to serve as a substrate for iRNA-directed cleavage. In one embodiment, the target sequence is within the protein-coding region of MAPT. The term "neurodegenerative disease associated with the MAPT gene" includes any disease or disorder associated with reduced MAPT expression and/or activity. Symptoms or signs usually include cognitive decline, memory impairment, loss of ability to understand or express language, behavioral abnormalities, impaired motor function, increased number or volume of neurofibrillary inclusion bodies. Typical "neurodegenerative diseases associated with the MAPT gene" include tauopathy, Alzheimer's disease (AD), frontotemporal dementia and parkinsonism linked to chromosome 17 (FTDP-17), frontotemporal dementia (FTD), progressive supranuclear palsy (PSP), corticobasal degeneration (CBD), chronic traumatic encephalopathy (CTE), epilepsy, Dravet syndrome (DS), Pick's disease (PiD), argyrophilic grain disease (AGD), and globular glial tauopathy (GGT).

As used herein, in the context of RNA-mediated gene silencing, the sense strand (also referred to as SS or SS strand) refers to a strand comprising a sequence identical or substantially identical to a target mRNA sequence; the antisense strand (also referred to as AS or AS strand) refers to a strand comprising a sequence complementary to a target mRNA sequence.

In the context describing the sense strand of the dsRNA described herein, the term "the sense strand comprises a sequence of at least 15 contiguous nucleotides differing from any one of the nucleotide sequences of SEQ ID NO: 1 to SEQ ID NO: 10 and SEQ ID NO: 21 to SEQ ID NO: 78 by no more than 3 nucleotides." is intended to mean that the sense strand of the dsRNA described herein comprises at least 15 contiguous nucleotides of any one of the sense strands set forth in SEQ ID NO: 1 to SEQ ID NO: 10 and SEQ ID NO: 21 to SEQ ID NO: 78, or a sequence differing from at least 15 contiguous nucleotides of any one of the sense strands set forth in SEQ ID NO: 1 to SEQ ID NO: 10 and SEQ ID NO: 21 to SEQ ID NO: 78 by no more than 3 nucleotides (optionally by no more than 2 nucleotides, optionally by 1 nucleotide, and optionally by 0 nucleotides). Other similar descriptions in the context of the present disclosure should also be understood in the same way.

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably. Unless otherwise specified, in the context of the present disclosure, "G", "C", "A", "T", and "U" represent nucleosides, and comprise the bases of guanine, cytosine, adenine, thymidine, and uracil, respectively. It is well-known to those skilled in the art that the mutual replacement between bases T and U does not significantly affect the properties of the dsRNA sequence. In the sequences of the present disclosure, U can be arbitrarily replaced with T, and the sequences after the replacement also fall within the protection scope of the present disclosure. In the sequences of the present disclosure, for the same nucleic acid strand, the direction from the 5' end to the 3' end is a direction from the left to the right; the lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; VP means that the 5' end of the nucleoside adjacent to the letters on the left/right side is a trans-vinylphosphodiester group (5'-*E*-VP); the lowercase letter s means that the two nucleosides adjacent to the letter s are linked by a phosphorothioate diester group; unless otherwise specified, two adjacent nucleosides are linked by a phosphodiester group. Unless otherwise specified, the "RNAi agent", "nucleotide", "compound", "chemical modification", "oligonucleotide", "double-stranded RNAi inhibitor molecule", "siRNA", "siRNA conjugate", "dsRNA", "nucleic acid", "RNAi", and "nucleoside" of the present disclosure can each independently be present in the form of a salt, a mixed salt, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts. When it is present in the form of a salt, part of the groups may be ionized to form anions/cations; for example, phosphodiester groups and phosphorothioate diester groups may be present in the form of anions, and the structures in the form of a salt corresponding to the following structures also fall within the protection scope of the present disclosure. Unless otherwise specified, the 3' position of the first nucleotide at the 3' end of each strand is hydroxy; the 5' position of the first nucleotide at the 5' end of each strand is hydroxy.

The modifications and linking groups described above separately have the structures shown in the table below, where Base represents a base:

**Table 1**

| Structure | Name |
|---|---|
| 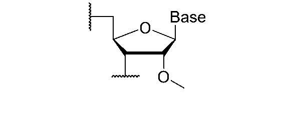 | 2'-Methoxy-modified nucleoside |
| 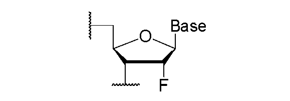 | 2'-Fluoro-modified nucleoside |
| 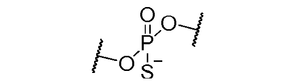 | Anion form of phosphorothioate diester group |
| 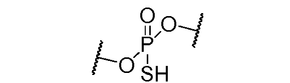 | Phosphorothioate diester group |
| 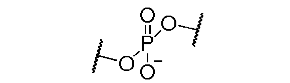 | Anion form of phosphodiester group |
| 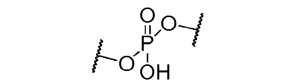 | Phosphodiester group |
| 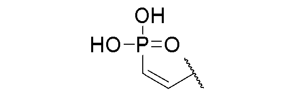 | Z-Vinylphosphoric acid group (Z-VP) |
| 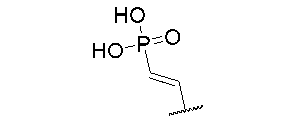 | E-Vinylphosphoric acid group (E-VP) |

The term "lipophilic group" or "lipophilic moiety" broadly refers to any compound or chemical moiety that has an affinity for lipids. One way to characterize the lipophilicity of a lipophilic moiety is by the octanol-water partition coefficient, logK_{ow}, where K_{ow} is the ratio of the concentration of a chemical substance in the octanol phase to its concentration in the aqueous phase in a biphasic system in equilibrium. In principle, a chemical substance is lipophilic when its logK_{ow} exceeds 0. Typically, a lipophilic moiety has a logK_{ow} of greater than 1, greater than 1.5, greater than 2, greater than 3, greater than 4, greater than 5, or greater than 10; for example, 6-aminohexanol has a logK_{ow} of about 0.7, and cholesteryl N-(hexan-6-ol)carbamate has a logK_{ow} of 10.7.

The lipophilicity of a molecule may vary relative to the functional groups it carries. For example, adding hydroxy or an amine group to an end of a lipophilic moiety may increase or decrease the partition coefficient (e.g., logK_{ow}) value of the lipophilic moiety. For example, a lipophilic moiety may be an aliphatic, cyclic such as alicyclic, or polycyclic such as polyalicyclic compounds, such as steroids (e.g., sterols) or linear or branched aliphatic hydrocarbons. A lipophilic moiety may generally contain a hydrocarbon chain, and the hydrocarbon chain may be cyclic or acyclic. The hydrocarbon chain may contain various substituents and/or one or more heteroatoms, such as oxygen or sulfur atoms. Such lipophilic aliphatic moieties include, but are not limited to, saturated or unsaturated C₄-C₃₀ hydrocarbons (e.g., C₁₀-C₃₀ hydrocarbons), saturated or unsaturated fatty acids, waxes (e.g., fatty acids and monohydric alcohol esters of fatty diamides), terpenes (e.g., C₁₀ terpenes, C₁₅ sesquiterpenes, C₂₀ diterpenes, C₃₀ triterpenes, and C₄₀ tetraterpenes), and other polyalicyclic hydrocarbons; for example, a lipophilic moiety may be an optionally substituted C₁₀₋₃₀ linear alkyl group; for example, a lipophilic moiety may be an optionally substituted C₁₄₋₂₄ linear alkyl group.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the dsRNA; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo^{®} Luciferase Assay System: the Firefly chemiluminescence value (Fir) and the Renilla chemiluminescence value (Ren) are each read, and the relative value Ratio = Ren/Fir is calculated; in the present disclosure, the ratio of the remaining mRNA expression level (or residual activity%) = Ratio (dsRNA-treated group)/Ratio (dsRNA-free control group), and the inhibition rate (%) = 100% - remaining mRNA expression level (%).

Unless otherwise specified, the "compound", "ligand", "nucleic acid-ligand conjugate", "nucleic acid", "conjugate", "chemical modification", "targeting ligand", "dsRNA", "siRNA", and "RNAi" of the present disclosure can each independently be present in the form of a salt, a mixed salt, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dose" refers to the amount of a drug, a compound, or a pharmaceutical composition necessary to obtain any one or more beneficial or desired therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

As used herein, "patient", "subject", and "individual" are used interchangeably and include human or non-human animals, e.g., mammals, e.g., humans or monkeys.

The dsRNA provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the dsRNA of the present disclosure using a nucleoside monomer with a corresponding modification. Methods of preparing a nucleoside monomer with a corresponding modification and introducing a modified nucleotide group into a dsRNA are also well known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt" and "blunt-ended" are used interchangeably and mean that there are no unpaired nucleotides or nucleotide analogs at a given end of a dsRNA, i.e., no nucleotide overhang. In most cases, a dsRNA whose both ends are blunt-ended will be double-stranded over its entire length.

The terms "about" and "approximately" mean that a numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprise" should be assumed to be within an acceptable margin of error for that specific value.

Unless otherwise specified, "optionally" or "optional" means that the event or circumstance subsequently described may, but does not necessarily, occur, and this description includes instances where the event or circumstance occurs or does not occur. For example, "optionally, R₁ and R₂ are directly linked to form a ring" means that R₁ and R₂ being directly linked to form a ring may occur, but does not necessarily exist, and this description includes an instance where R₁ and R₂ are directly linked to form a ring and an instance where R₁ and R₂ do not form a ring. In the chemical structural formulas of the present disclosure, " 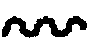 " or 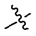 may be linked to any group or groups in accordance with the scope of the invention described herein.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C₁₋C₆ alkyl, C₂-C₆ alkenyl, C₂-C₆ alkynyl, cyano, hydroxy, oxo, carboxyl, cycloalkyl, cycloalkenyl, heterocyclyl, heteroaryl, aryl, ketones, alkoxycarbonyl, aryloxycarbonyl, heteroaryloxycarbonyl, or halogens (e.g., F, Cl, Br, and I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a group of a plurality of different substituents.

### DETAILED DESCRIPTION

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Design of Human MAPT dsRNAs

With the human MAPT gene (NM_016841.4) as a target gene, dsRNAs of 19/21nt were designed in accordance with the general rules for active dsRNAs. The sequences of unmodified sense and antisense strands are shown in Tables 2A and 2B, and the sequences of modified sense and antisense strands are detailed in Table 3.

**Table 2A. The unmodified sense and antisense strands of dsRNAs of the present disclosure**

| Double strand No. | SEQ ID NO: | Sense strand (5'-3') | SEQ ID NO: | Antisense strand (5'-3') |
|---|---|---|---|---|
| TJR103374 | 1 | GGGCGGAGAUCGUGUACAA | 11 | UUGUACACGAUCUCCGCCCCG |
| TJR102156 | 2 | GGCGGAGGAAAUAAAAG A | 12 | UCUUUUUAUUUCCUCCGCCAG |
| TJR103600 | 3 | GGGACUGGAAGCGAUGACA | 13 | UGUCAUCGCUUCCAGUCCCGU |
| TJR103599 | 4 | GACUGGAAGCGAUGACAAA | 14 | UUUGUCAUCGCUUCCAGUCCC |
| TJR102157 | 5 | GCGGAGGAAAUAAAAAGA U | 15 | AUCUUUUUAUUUCCUCCGCCA |
| TJR103604 | 6 | AAAAAGAUUGAAACCCACA | 16 | UGUGGGUUUCAAUCUUUUUAU |
| TJR103603 | 7 | GCUACACCAUGCACCAAGA | 17 | UCUUGGUGCAUGGUGUAGCCC |
| TJR103601 | 8 | CCAGACCUGAAGAAUGUCA | 18 | UGACAUUCUUCAGGUCUGGCA |
| TJR103605 | 9 | CAGACCUGAAGAAUGUCAA | 19 | UUGACAUUCUUCAGGUCUGGC |
| TJR103602 | 10 | CUGAAGAAUGUCAAGUCCA | 20 | UGGACUUGACAUUCUUCAGGU |
| TJR103375 | 77 | GAGAUCGUGUACAAGUCGC CA | 135 | UGGCGACUUGUACACGAUCUC CG |
| TJR103376 | 78 | GGCGGAGAUCGUGUACAA | 136 | UUGUACACGAUCUCCGCC |
| TJR103646 | 287 | GGCGGAGAUCGUGUACAAC | 288 | UUUGUACACGAUCUCCGCC |

**Table 2B. The sense and antisense strands of dsRNAs of the present disclosure**

| SEQ ID NO: | Sense strand (5'-3') | SEQ ID NO: | Antisense strand (5'-3') |
|---|---|---|---|
| 21 | CCACCAGGAUUCCAGCAAA | 79 | UUUGCUGGAAUCCUGGUGGCG |
| 22 | CGAUGACAAAAAAGCCAAA | 80 | UUUGGCUUUUUUGUCAUCGCU |
| 23 | GGACACGUCUCCACGGCAU | 81 | AUGCCGUGGAGACGUGUCCCC |
| 24 | GGGGGACAGGAAAGAUCAA | 82 | UUGAUCUUUCCUGUCCCCCAA |
| 25 | CCUGGAAGACGAAGCUGCU | 83 | AGCAGCUUCGUCUUCCAGGCU |
| 26 | GGACUGGAAGCGAUGACAA | 84 | UUGUCAUCGCUUCCAGUCCCG |
| 27 | CUGGAAGCGAUGACAAAAA | 85 | UUUUUGUCAUCGCUUCCAGUC |
| 28 | GGAAGCGAUGACAAAAAAA | 86 | UUUUUUUGUCAUCGCUUCCAG |
| 29 | CCAUGCCAGACCUGAAGAA | 87 | UUCUUCAGGUCUGGCAUGGGC |
| 30 | AAAAGAUUGAAACCCACAA | 88 | UUGUGGGUUUCAAUCUUUUUA |
| 31 | GCGAGAACGCCAAAGCCAA | 89 | UUGGCUUUGGCGUUCUCGCGG |
| 32 | CCUCCAUGUAGAAGAGGGA | 90 | UCCCUCUUCUACAUGGAGGGG |
| 33 | CCAUGUAGAAGAGGGAGAA | 91 | UUCUCCCUCUUCUACAUGGAG |
| 34 | GUGUGUUCGUGGAGCCACA | 92 | UGUGGCUCCACGAACACACCA |
| 35 | GGGAGACGUCCACCCGUUU | 93 | AAACGGGUGGACGUCUCCCAA |
| 36 | CACCAGGAUUCCAGCAAAA | 94 | UUUUGCUGGAAUCCUGGUGGC |
| 37 | CGCACCCCGUCCCUUCCAA | 95 | UUGGAAGGGACGGGGUGCGGG |
| 38 | GCCUCUUGGGAGACGUCCA | 96 | UGGACGUCUCCCAAGAGGCAC |
| 39 | GCCUAUACCCCUCAUCACA | 97 | UGUGAUGAGGGGUAUAGGCAG |
| 40 | GCACAAGAGUGGGACCCCA | 98 | UGGGGUCCCACUCUUGUGCCU |
| 41 | CCCCAGCCUGGAAGACGAA | 99 | UUCGUCUUCCAGGCUGGGGGU |
| 42 | GCGAUGACAAAAAAGCCAA | 100 | UUGGCUUUUUUGUCAUCGCUU |
| 43 | GCCACCAGGAUUCCAGCAA | 101 | UUGCUGGAAUCCUGGUGGCGU |
| 44 | CCUGGCGGAGGAAAUAAAA | 102 | UUUUAUUUCCUCCGCCAGGGA |
| 45 | CUGGCGGAGGAAAUAAAAA | 103 | UUUUUAUUUCCUCCGCCAGGG |
| 46 | GCGGAGGAAAUAAAAAGAA | 104 | UUCUUUUUAUUUCCUCCGCCA |
| 47 | AGCUGACCUUCCGCGAGAA | 105 | UUCUCGCGGAAGGUCAGCUUG |
| 48 | CCUUCCGCGAGAACGCCAA | 106 | UUGGCGUUCUCGCGGAAGGUC |
| 49 | CUUCCGCGAGAACGCCAAA | 107 | UUUGGCGUUCUCGCGGAAGGU |
| 50 | CGCGAGAACGCCAAAGCCA | 108 | UGGCUUUGGCGUUCUCGCGGA |
| 51 | GGGGUGUCACAGAGGCAGU | 109 | ACUGCCUCUGUGACACCCCCA |
| 52 | GACGUCCACCCGUUUCCAA | 110 | UUGGAAACGGGUGGACGUCUC |
| 53 | GGGCACUGCUCAGCUGUGA | 111 | UCACAGCUGAGCAGUGCCCUG |
| 54 | GCUCAGCUCCACAUGCAUA | 112 | UAUGCAUGUGGAGCUGAGCAG |
| 55 | GGAACACACCCCCUUGGAA | 113 | UUCCAAGGGGGUGUGUUCCCC |
| 56 | CUGAGAACCUGAAGCACCA | 114 | UGGUGCUUCAGGUUCUCAGUG |
| 57 | GGAACUGCUGCCAUGAUUU | 115 | AAAUCAUGGCAGCAGUUCCAA |
| 58 | CUGGGACCCUCACCACGAA | 116 | UUCGUGGUGAGGGUCCCAGUU |
| 59 | GCCAGAAGGGCCAGGCCAA | 117 | UUGGCCUGGCCCUUCUGGCCU |
| 60 | GGCCAACGCCACCAGGAUU | 118 | AAUCCUGGUGGCGUUGGCCUG |
| 61 | AAAACAUGGCCACAUCCAA | 119 | UUGGAUGUGGCCAUGUUUUUG |
| 62 | GAACUGCUGCCAUGAUUUU | 120 | AAAAUCAUGGCAGCAGUUCCA |
| 63 | CCUUCUCAGUAAUGACCCU | 121 | AGGGUCAUUACUGAGAAGGGG |
| 64 | CAUCCAACUGGGACCCUCA | 122 | UGAGGGUCCCAGUUGGAUGAG |
| 65 | CCCUCACCACGAAUCUCAU | 123 | AUGAGAUUCGUGGUGAGGGUC |
| 66 | CCGUCACAGAUGUGAGCCA | 124 | UGGCUCACAUCUGUGACGGGA |
| 67 | GAACACACCCCCUUGGAAA | 125 | UUUCCAAGGGGGUGUGUUCCC |
| 68 | GGCAGUGGUCCGUACUCCA | 126 | UGGAGUACGGACCACUGCCAC |
| 69 | CGAAGAUUGGGUCCCUGGA | 127 | UCCAGGGACCCAAUCUUCGAC |
| 70 | GGUUGGAACUGCUGCCAUA | 128 | UAUGGCAGCAGUUCCAACCUU |
| 71 | GGUACCUACUCCAUACUGA | 129 | UCAGUAUGGAGUAGGUACCUC |
| 72 | AGAUUGGGUCCCUGGACAA | 130 | UUGUCCAGGGACCCAAUCUUC |
| 73 | GGGUCCCUGGACAAUAUCA | 131 | UGAUAUUGUCCAGGGACCCAA |
| 74 | AGAAGGAGAGGCUCUGAAA | 132 | UUUCAGAGCCUCUCCUUCUCC |
| 75 | CCCUGUUGUGGGGGUGUCA | 133 | UGACACCCCCACAACAGGGCC |
| 76 | CUGUUGUGGGGGUGUCACA | 134 | UGUGACACCCCCACAACAGGG |

**Table 3. The sense and antisense strands of dsRNAs of the present disclosure**

| Double strand No. | SEQ ID NO: | SS strand (5'-3') | SEQ ID NO: | AS strand (5'-3') |
|---|---|---|---|---|
| TJR10 1212 | 137 | GmsGmsCmGmGmAmGfGfAfAmAmU mAmAmAmAmAmGmAm | 202 | VPUmsCfsUmUmUmUfUmAmU mUmUmCfCmUfCmCfGmCmCm sAmsGm |
| TJR10 1225 | 138 | GmsGmsGmAmCmUmGfGfAfAmGmC mGmAmUmGmAmCmAm | 203 | VPUmsGfsUmCmAmUfCmGmC mUmUmCfCmAfGmUfCmCmCm sGmsUm |
| TJR10 1226 | 139 | GmsAmsCmUmGmGmAfAfGfCmGmA mUmGmAmCmAmAmAm | 204 | VPUmsUfsUmGmUmCfAmUmC mGmCmUfUmCfCmAfGmUmCm sCmsCm |
| TJR10 1231 | 140 | GmsCmsGmGmAmGmGfAfAfAmUmA mAmAmAmAmGmAmAm | 205 | VPUmsUfsCmUmUmUfUmUmA mUmUmUfCmCfUmCfCmGmCm sCmsAm |
| TJR10 1232 | 141 | AmsAmsAmAmAmGmAfUfUfGmAmA mAmCmCmCmAmCmAm | 206 | VPUmsGfsUmGmGmGfUmUmU mCmAmAfUmCfUmUfUmUmUm sAmsUm |
| TJR10 1242 | 142 | GmsCmsUmAmCmAmCfCfAfUmGmC mAmCmCmAmAmGmAm | 207 | VPUmsCfsUmUmGmGfUmGmC mAmUmGfGmUfGmUfAmGmC msCmsCm |
| TJR10 1248 | 143 | CmsCmsAmGmAmCmCfUfGfAmAmG mAmAmUmGmUmCmAm | 208 | VPUmsGfsAmCmAmUfUmCmU mUmCmAfGmGfUmCfUmGmGm sCmsAm |
| TJR10 1258 | 144 | GmsGmsGmCmGmGmAfGfAfUmCmG mUmGmUmAmCmAmAm | 209 | VPUmsUfsGmUmAmCfAmCmG mAmUmCfUmCfCmGfCmCmCm sCmsGm |
| TJR10 1261 | 145 | CmsAmsGmAmCmCmUfGfAfAmGmA mAmUmGmUmCmAmAm | 210 | VPUmsUfsGmAmCmAfUmUmC mUmUmCfAmGfGmUfCmUmGm sGmsCm |
| TJR10 1262 | 146 | CmsUmsGmAmAmGmAfAfUfGmUmC mAmAmGmUmCmCmAm | 211 | VPUmsGfsGmAmCmUfUmGmA mCmAmUfUmCfUmUfCmAmGm sGmsUm |
| TJR10 1203 | 147 | CmsCmsAmCmCmAmGfGfAfUmUmC mCmAmGmCmAmAmAm | 212 | VPUmsUfsUmGmCmUfGmGmA mAmUmCfCmUfGmGfUmGmGm sCmsGm |
| TJR10 1204 | 148 | CmsGmsAmUmGmAmCfAfAfAmAmA mAmGmCmCmAmAmAm | 213 | VPUmsUfsUmGmGmCfUmUmU mUmUmUfGmUfCmAfUmCmGm sCmsUm |
| TJR10 1205 | 149 | GmsGmsAmCmAmCmGfUfCfUmCmC mAmCmGmGmCmAmAm | 214 | VPUmsUfsGmCmCmGfUmGmG mAmGmAfCmGfUmGfUmCmCm sCmsCm |
| TJR10 1206 | 150 | GmsGmsGmGmGmAmCfAfGfGmAmA mAmGmAmUmCmAmAm | 215 | VPUmsUfsGmAmUmCfUmUmU mCmCmUfGmUfCmCfCmCmCm sAmsAm |
| TJR10 1207 | 151 | CmsCmsUmGmGmAmAfGfAfCmGmA mAmGmCmUmGmCmAm | 216 | VPUmsGfsCmAmGmCfUmUmC mGmUmCfUmUfCmCfAmGmGm sCmsUm |
| TJR10 1208 | 152 | GmsGmsAmCmUmGmGfAfAfGmCmG mAmUmGmAmCmAmAm | 217 | VPUmsUfsGmUmCmAfUmCmG mCmUmUfCmCfAmGfUmCmCm sCmsGm |
| TJR10 1209 | 153 | CmsUmsGmGmAmAmGfCfGfAmUmG mAmCmAmAmAmAmAm | 218 | VPUmsUfsUmUmUmGfUmCmA mUmCmGfCmUfUmCfCmAmGm sUmsCm |
| TJR10 1210 | 154 | GmsGmsAmAmGmCmGfAfUfGmAmC mAmAmAmAmAmAmAm | 219 | VPUmsUfsUmUmUmUfUmGmU mCmAmUfCmGfCmUfUmCmCm sAmsGm |
| TJR10 1211 | 155 | CmsCmsAmUmGmCmCfAfGfAmCmC mUmGmAmAmGmAmAm | 220 | VPUmsUfsCmUmUmCfAmGmG mUmCmUfGmGfCmAfUmGmGm sGmsCm |
| TJR10 1213 | 156 | AmsAmsAmAmGmAmUfUfGfAmAmA mCmCmCmAmCmAmAm | 221 | VPUmsUfsGmUmGmGfGmUmU mUmCmAfAmUfCmUfUmUmUm sUmsAm |
| TJR10 1214 | 157 | GmsCmsGmAmGmAmAfCfGfCmCmA mAmAmGmCmCmAmAm | 222 | VPUmsUfsGmGmCmUfUmUmG mGmCmGfUmUfCmUfCmGmCm sGmsGm |
| TJR10 1215 | 158 | CmsCmsUmCmCmAmUfGfUfAmGmA mAmGmAmGmGmGmAm | 223 | VPUmsCfsCmCmUmCfUmUmC mUmAmCfAmUfGmGfAmGmG msGmsGm |
| TJR10 1216 | 159 | CmsCmsAmUmGmUmAfGfAfAmGmA mGmGmGmAmGmAmAm | 224 | VPUmsUfsCmUmCmCfCmUmC mUmUmCfUmAfCmAfUmGmGm sAmsGm |
| TJR10 1217 | 160 | GmsUmsGmUmGmUmUfCfGfUmGmG mAmGmCmCmAmCmAm | 225 | VPUmsGfsUmGmGmCfUmCmC mAmCmGfAmAfCmAfCmAmCm sCmsAm |
| TJR10 1218 | 161 | GmsGmsGmAmGmAmCfGfUfCmCmA mCmCmCmGmUmUmAm | 226 | VPUmsAfsAmCmGmGfGmUmG mGmAmCfGmUfCmUfCmCmCm sAmsAm |
| TJR10 1219 | 162 | CmsAmsCmCmAmGmGfAfUfUmCmC mAmGmCmAmAmAmAm | 227 | VPUmsUfsUmUmGmCfUmGmG mAmAmUfCmCfUmGfGmUmGm sGmsCm |
| TJR10 1220 | 163 | CmsGmsCmAmCmCmCfCfGfUmCmC mCmUmUmCmCmAmAm | 228 | VPUmsUfsGmGmAmAfGmGmG mAmCmGfGmGfGmUfGmCmGm sGmsGm |
| TJR10 1221 | 164 | GmsCmsCmUmCmUmUfGfGfGmAmG mAmCmGmUmCmCmAm | 229 | VPUmsGfsGmAmCmGfUmCmU mCmCmCfAmAfGmAfGmGmCm sAmsCm |
| TJR10 1222 | 165 | GmsCmsCmUmAmUmAfCfCfCmCmU mCmAmUmCmAmCmAm | 230 | VPUmsGfsUmGmAmUfGmAmG mGmGmGfUmAfUmAfGmGmC msAmsGm |
| TJR10 1223 | 166 | GmsCmsAmCmAmAmGfAfGfUmGmG mGmAmCmCmCmCmAm | 231 | VPUmsGfsGmGmGmUfCmCmC mAmCmUfCmUfUmGfUmGmCm sCmsUm |
| TJR10 1224 | 167 | CmsCmsCmCmAmGmCfCfUfGmGmA mAmGmAmCmGmAmAm | 232 | VPUmsUfsCmGmUmCfUmUmC mCmAmGfGmCfUmGfGmGmGm sGmsUm |
| TJR10 1227 | 168 | GmsCmsGmAmUmGmAfCfAfAmAmA mAmAmGmCmCmAmAm | 233 | VPUmsUfsGmGmCmUfUmUmU mUmUmGfUmCfAmUfCmGmCm sUmsUm |
| TJR10 1228 | 169 | GmsCmsCmAmCmCmAfGfGfAmUmU mCmCmAmGmCmAmAm | 234 | VPUmsUfsGmCmUmGfGmAmA mUmCmCfUmGfGmUfGmGmCm sGmsUm |
| TJR10 1229 | 170 | CmsCmsUmGmGmCmGfGfAfGmGmA mAmAmUmAmAmAmAm | 235 | VPUmsUfsUmUmAmUfUmUmC mCmUmCfCmGfCmCfAmGmGm sGmsAm |
| TJR10 1230 | 171 | CmsUmsGmGmCmGmGfAfGfGmAmA mAmUmAmAmAmAmAm | 236 | VPUmsUfsUmUmUmAfUmUmU mCmCmUfCmCfGmCfCmAmGm sGmsGm |
| TJR10 1233 | 172 | AmsGmsCmUmGmAmCfCfUfUmCmC mGmCmGmAmGmAmAm | 237 | VPUmsUfsCmUmCmGfCmGmG mAmAmGfGmUfCmAfGmCmUm sUmsGm |
| TJR10 1234 | 173 | CmsCmsUmUmCmCmGfCfGfAmGmA mAmCmGmCmCmAmAm | 238 | VPUmsUfsGmGmCmGfUmUmC mUmCmGfCmGfGmAfAmGmGm sUmsCm |
| TJR10 1235 | 174 | CmsUmsUmCmCmGmCfGfAfGmAmA mCmGmCmCmAmAmAm | 239 | VPUmsUfsUmGmGmCfGmUmU mCmUmCfGmCfGmGfAmAmGm sGmsUm |
| TJR10 1236 | 175 | CmsGmsCmGmAmGmAfAfCfGmCmC mAmAmAmGmCmCmAm | 240 | VPUmsGfsGmCmUmUfUmGmG mCmGmUfUmCfUmCfGmCmGm sGmsAm |
| TJR101237 | 176 | GmsGmsGmGmUmGmUfCfAfCmAmG mAmGmGmCmAmGmAm | 241 | VPUmsCfsUmGmCmCfUmCmU mGmUmGfAmCfAmCfCmCmCm sCmsAm |
| TJR10 1238 | 177 | GmsAmsCmGmUmCmCfAfCfCmCmG mUmUmUmCmCmAmAm | 242 | VPUmsUfsGmGmAmAfAmCmG mGmGmUfGmGfAmCfGmUmCm sUmsCm |
| TJR10 1239 | 178 | GmsGmsGmCmAmCmUfGfCfUmCmA mGmCmUmGmUmGmAm | 243 | VPUmsCfsAmCmAmGfCmUmG mAmGmCfAmGfUmGfCmCmCm sUmsGm |
| TJR10 1240 | 179 | GmsCmsUmCmAmGmCfUfCfCmAmC mAmUmGmCmAmUmAm | 244 | VPUmsAfsUmGmCmAfUmGmU mGmGmAfGmCfUmGfAmGmCm sAmsGm |
| TJR10 1241 | 180 | GmsGmsAmAmCmAmCfAfCfCmCmC mCmUmUmGmGmAmAm | 245 | VPUmsUfsCmCmAmAfGmGmG mGmGmUfGmUfGmUfUmCmCm sCmsCm |
| TJR10 1243 | 181 | CmsUmsGmAmGmAmAfCfCfUmGmA mAmGmCmAmCmCmAm | 246 | VPUmsGfsGmUmGmCfUmUmC mAmGmGfUmUfCmUfCmAmGm sUmsGm |
| TJR10 1244 | 182 | GmsGmsAmAmCmUmGfCfUfGmCmC mAmUmGmAmUmUmAm | 247 | VPUmsAfsAmUmCmAfUmGmG mCmAmGfCmAfGmUfUmCmCm sAmsAm |
| TJR10 1245 | 183 | CmsUmsGmGmGmAmCfCfCfUmCmA mCmCmAmCmGmAmAm | 248 | VPUmsUfsCmGmUmGfGmUmG mAmGmGfGmUfCmCfCmAmGm sUmsUm |
| TJR10 1246 | 184 | GmsCmsCmAmGmAmAfGfGfGmCmC mAmGmGmCmCmAmAm | 249 | VPUmsUfsGmGmCmCfUmGmG mCmCmCfUmUfCmUfGmGmCm sCmsUm |
| TJR10 1247 | 185 | GmsGmsCmCmAmAmCfGfCfCmAmC mCmAmGmGmAmUmAm | 250 | VPUmsAfsUmCmCmUfGmGmU mGmGmCfGmUfUmGfGmCmCm sUmsGm |
| TJR10 1249 | 186 | AmsAmsAmAmCmAmUfGfGfCmCmA mCmAmUmCmCmAmAm | 251 | VPUmsUfsGmGmAmUfGmUmG mGmCmCfAmUfGmUfUmUmUm sUmsGm |
| TJR10 1250 | 187 | GmsAmsAmCmUmGmCfUfGfCmCmA mUmGmAmUmUmUmAm | 252 | VPUmsAfsAmAmUmCfAmUmG mGmCmAfGmCfAmGfUmUmCm sCmsAm |
| TJR10 1251 | 188 | CmsCmsUmUmCmUmCfAfGfUmAmA mUmGmAmCmCmCmAm | 253 | VPUmsGfsGmGmUmCfAmUmU mAmCmUfGmAfGmAfAmGmG msGmsGm |
| TJR10 1252 | 189 | CmsAmsUmCmCmAmAfCfUfGmGmG mAmCmCmCmUmCmAm | 254 | VPUmsGfsAmGmGmGfUmCmC mCmAmGfUmUfGmGfAmUmG msAmsGm |
| TJR10 1253 | 190 | CmsCmsCmUmCmAmCfCfAfCmGmA mAmUmCmUmCmAmAm | 255 | VPUmsUfsGmAmGmAfUmUmC mGmUmGfGmUfGmAfGmGmG msUmsCm |
| TJR10 1254 | 191 | CmsCmsGmUmCmAmCfAfGfAmUmG mUmGmAmGmCmCmAm | 256 | VPUmsGfsGmCmUmCfAmCmA mUmCmUfGmUfGmAfCmGmGm sGmsAm |
| TJR10 1255 | 192 | GmsAmsAmCmAmCmAfCfCfCmCmC mUmUmGmGmAmAmAm | 257 | VPUmsUfsUmCmCmAfAmGmG mGmGmGfUmGfUmGfUmUmC msCmsCm |
| TJR10 1256 | 193 | GmsGmsCmAmGmUmGfGfUfCmCmG mUmAmCmUmCmCmAm | 258 | VPUmsGfsGmAmGmUfAmCmG mGmAmCfCmAfCmUfGmCmCm sAmsCm |
| TJR10 1257 | 194 | CmsGmsAmAmGmAmUfUfGfGmGmU mCmCmCmUmGmGmAm | 259 | VPUmsCfsCmAmGmGfGmAmC mCmCmAfAmUfCmUfUmCmGm sAmsCm |
| TJR10 1259 | 195 | GmsGmsUmUmGmGmAfAfCfUmGmC mUmGmCmCmAmUmAm | 260 | VPUmsAfsUmGmGmCfAmGmC mAmGmUfUmCfCmAfAmCmCm sUmsUm |
| TJR10 1260 | 196 | GmsGmsUmAmCmCmUfAfCfUmCmC mAmUmAmCmUmGmAm | 261 | VPUmsCfsAmGmUmAfUmGmG mAmGmUfAmGfGmUfAmCmCm sUmsCm |
| TJR10 1263 | 197 | AmsGmsAmUmUmGmGfGfUfCmCmC mUmGmGmAmCmAmAm | 262 | VPUmsUfsGmUmCmCfAmGmG mGmAmCfCmCfAmAfUmCmUm sUmsCm |
| TJR10 1264 | 198 | GmsGmsGmUmCmCmCfUfGfGmAmC mAmAmUmAmUmCmAm | 263 | VPUmsGfsAmUmAmUfUmGmU mCmCmAfGmGfGmAfCmCmCm sAmsAm |
| TJR10 1265 | 199 | AmsGmsAmAmGmGmAfGfAfGmGmC mUmCmUmGmAmAmAm | 264 | VPUmsUfsUmCmAmGfAmGmC mCmUmCfUmCfCmUfUmCmUm sCmsCm |
| TJR10 1266 | 200 | CmsCmsCmUmGmUmUfGfUfGmGmG mGmGmUmGmUmCmAm | 265 | VPUmsGfsAmCmAmCfCmCmC mCmAmCfAmAfCmAfGmGmGm sCmsCm |
| TJR10 1267 | 201 | CmsUmsGmUmUmGmUfGfGfGmGmG mUmGmUmCmAmCmAm | 266 | VPUmsGfsUmGmAmCfAmCmC mCmCmCfAmCfAmAfCmAmGm sGmsGm |
| TJR10 2242 | 267 | GhdsGmsGmCmGmGmAfGfAfUmCmG mUmGmUmAmCmsAmsAm | 277 | VPUmsUfsGmUfAmCfAmCmGm AfUmCfUmCfCmGfCmCfCmsCm sGm |
| TJR10 3670 | 268 | GhdsAmsCmUmGmGmAfAfGfCmGmA mUmGmAmCmAmsAmsAm | 278 | VPUmsUfsUmGfUmCfAmUmCm GfCmUfUmCfCmAfGmUfCmsC msCm |
| TJR10 2182 | 269 | GhdsGmsGmAmCmUmGfGfAfAmGmC mGmAmUmGmAmsCmsAm | 279 | VPUmsGfsUmCfAmUfCmGmCm UfUmCfCmAfGmUfCmCfCmsG msUm |
| TJR10 3671 | 270 | GhdsGmsCmGmGmAmGfGfAfAmAmU mAmAmAmAmAmsGmsAm | 280 | VPUmsCfsUmUfUmUfUmUfUmAmUm UfUmCfCmUfCmCfGmCfCmsAm sGm |
| TJR10 3672 | 271 | ChdsCmsAmGmAmCmCfUfGfAmAmG mAmAmUmGmUmsCmsAm | 281 | VPUmsGfsAmCfAmUfUmCmUm UfCmAfGmGfUmCfUmGfGmsC msAm |
| TJR10 3673 | 272 | ChdsUmsGmAmAmGmAfAfUfGmUmC mAmAmGmUmCmsCmsAm | 282 | VPUmsGfsGmAfCmUfUmGmAm CfAmUfUmCfUmUfCmAfGmsG msUm |
| TJR10 3674 | 273 | GhdsCmsUmAmCmAmCfCfAfUmGmC mAmCmCmAmAmsGmsAm | 283 | VPUmsCfsUmUfGmGfUmGmCm AfUmGfGmUfGmUfAmGfCmsC msCm |
| TJR10 3675 | 274 | AhdsAmsAmAmAmGmAfUfUfGmAmA mAmCmCmCmAmsCmsAm | 284 | VPUmsGfsUmGfGmGfUmUmUm CfAmAfUmCfUmUfUmUfUmsA msUm |
| TJR10 3676 | 275 | ChdsAmsGmAmCmCmUfGfAfAmGmA mAmUmGmUmCmsAmsAm | 285 | VPUmsUfsGmAfCmAfUmUmCm UfUmCfAmGfGmUfCmUfGmsG msCm |
| TJR10 3677 | 276 | GhdsCmsGmGmAmGmGfAfAfAmUmA mAmAmAmAmGmsAmsUm | 286 | VPAmsUfsCmUfUmUfUmUmAm UfUmUfCmCfUmCfCmGfCmsCm sAm |

In Tables 2A, 2B, and 3 above, the sequences are shown in the direction from the 5' end to the 3' end (left to right); "G", "C", "A", "T", and "U" represent nucleosides, and comprise the bases of guanine, cytosine, adenine, thymidine, and uracil, respectively. The lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; VP means that the 5' end of the nucleoside adjacent to the letters on the left/right side is a trans-vinylphosphodiester group (5'-*E*-VP); the lowercase letters hd mean that the nucleoside adjacent to the letters hd on the left side is a nucleoside with -O-n-hexadecyl linked to the 2' position of the sugar ring; the lowercase letter s means that the two nucleosides adjacent to the letter s are linked by a phosphorothioate diester group; unless otherwise specified, two adjacent nucleosides are linked by a phosphodiester group. Unless otherwise specified, the 3' position of the first nucleotide at the 3' end of each strand is hydroxy; the 5' position of the first nucleotide at the 5' end of each strand is hydroxy.

The structures of the 2'-methoxy-modified nucleoside, the 2'-fluoro-modified nucleoside, the nucleoside with -O-n-hexadecyl linked to the 2'-position of the sugar ring, the trans-vinylphosphodiester group (5'-E-VP), the phosphorothioate diester group, and the phosphodiester group are shown in the table below. When the dsRNAs of the present disclosure are present in the form of a salt, for example, in the form of a sodium salt, the structures of the salt forms corresponding to the structures in Table 4 below also fall within the protection scope of the present disclosure. In Table 4, Base represents a base at the corresponding position:

**Table 4**

| Structure | Name |
|---|---|
| 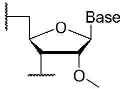 | 2'-Methoxy-modified nucleoside |
| 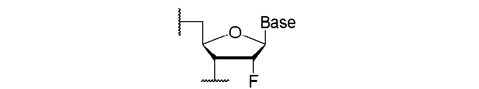 | 2'-Fluoro-modified nucleoside |
| 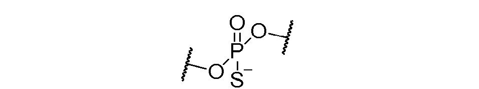 | Anion form of phosphorothioate diester group |
| 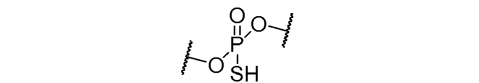 | Phosphorothioate diester group |
| 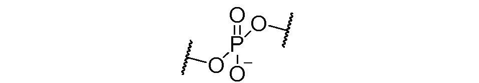 | Anion form of phosphodiester group |
| 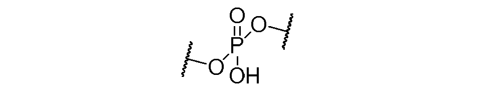 | Phosphodiester group |
| 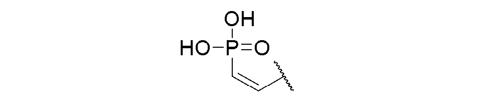 | Z-Vinylphosphoric acid group (Z-VP) |
| 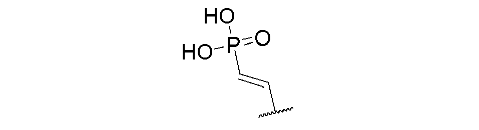 | E-Vinylphosphoric acid group (E-VP) |
| 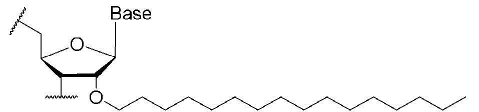 | Nucleoside with -O-n-hexadecyl linked to 2' position of sugar ring |

### Example 2. Synthesis of dsRNAs

The synthesis of dsRNAs does not differ from the conventional phosphoramidite solid-phase synthesis method. The synthesis process is briefly described below: Nucleoside phosphoramidite monomers were linked one by one according to a synthesis program on a Dr. Oligo48 synthesizer (Biolytic), with a universal CPG support as a start. The nucleoside monomer starting materials, nucleoside phosphoramidite monomers such as 2'-methoxy-modified nucleoside phosphoramidite monomers, 2'-fluoro-modified nucleoside phosphoramidite monomers, E-vinylphosphoric acid group-containing nucleoside phosphoramidite monomers, and nucleoside phosphoramidite monomers with -O-n-hexadecyl linked to the 2' position of the sugar ring, were purchased from Shanghai Hongene, Suzhou Genepharma, or Jiangsu Synthgene. 5-Ethylthio-1H-tetrazole (ETT) was used as an activator (0.6 M in acetonitrile), a 0.22 M solution of PADS in acetonitrile and collidine (1:1 by volume) (Suzhou Kroma) was used as a sulfurizing agent, and an iodopyridine/water solution (Kroma) was used as an oxidant.

After solid-phase synthesis was complete, the oligoribonucleotides were cleaved from the solid support by soaking in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by C18 reversed-phase chromatography with 0.1 M TEAA and acetonitrile as the mobile phase, and DMTr was removed using a 3% trifluoroacetic acid solution. The target oligonucleotides were collected, then lyophilized, identified as the target products by LC-MS, and quantified by UV (260 nm).

The single-stranded oligonucleotides obtained were complementarily paired in an equimolar ratio and annealed. The final dsRNAs were dissolved in 1× PBS, and the concentration of the solutions was adjusted to the concentration required for the experiment so that they were ready for use.

### Example 3. Inhibition of Human MAPT in MCF7 Cells by dsRNAs - Inhibitory Activity at 3 Concentration Points

The dsRNAs were subjected to *in vitro* molecular-level activity screening in MCF7 cells using 3 concentration gradients (10 nM, 1 nM, and 0.1 nM).

MCF7 cells were cultured at 37 °C with 5% CO2 in a Dulbecco's modified eagle medium containing 10% fetal bovine serum. 24 h prior to transfection, the MCF7 cells were seeded into a 96-well plate at a density of 1 × 10⁴ cells per well, with each well containing 100 µL of the culture medium.

The cells were transfected with the dsRNAs using Lipofectamine RNAi MAX (ThermoFisher, 13778150) according to the product instructions. The final gradient concentrations of the dsRNAs for transfection were 10 nM, 1 nM, and 0.1 nM, and two duplicate wells were set for each concentration. 48 h after treatment, total cell RNA extraction was performed using an FG0417-L/FG0418-XL high-throughput cell RNA extraction kit (FireGen, magnetic bead method), and RNA reverse transcription (Takara, RR037A) and quantitative real-time PCR (Thermo, 4444557) detection were performed to determine the mRNA level of human MAPT. The mRNA level of human MAPT was corrected based on the GAPDH internal reference gene level.

The instruments involved in the experiment are shown in Table 5.

**Table 5. Experimental instruments**

| Name | Company | Cat. No./model |
|---|---|---|
| Nanodrop | Thermo | Nanodrop One |
| qPCR system | ABI | 7500 Fast |
| PCR | Life | Pro-Flex PCR system |

For the real-time quantitative PCR detection, a probe Q-PCR detection experiment was used. Information on the primers is shown in Table 6.

**Table 6. Taqman primers**

| Primer name | SEQ ID NO: | Primer sequence (5'-3') |
|---|---|---|
| hMAPT-Forward | 289 | CCTAGAGCCTCACCTCCTAATA |
| hMAPT-Reverse | 290 | CACCCTCAGAATTACCGAAGAA |
| hMAPT-Probe | 291 | (6-FAM)- TGAGTTTGCCATGTTGAGCAGGACTAT- (BHQ1) |
| hGAPDH-Forward | 292 | GACCCCTTCATTGACCTCAACTAC |
| hGAPDH-Reverse | 293 | TTGACGGTGCCATGGAATTT |
| hGAPDH-Probe | 294 | (VIC)-TTACATGTTCCAATATGATTCC-(BHQ1) |

### Results analysis method:

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%.

The results are expressed relative to the remaining percentage of human MAPT mRNA expression in cells treated with the dsRNAs. The results in Table 7 show that the dsRNAs of the present disclosure all exhibited good inhibitory activity against MAPT mRNA.

**Table 7. The single-dose inhibitory activity of dsRNAs in MCF7 cells**

| Double strand No. | 10 nM expression remaining level (%) | STDEV (%) | 1 nM expression remaining level (%) | STDEV (%) | 0.1 nM expression remaining level (%) | STDEV (%) |
|---|---|---|---|---|---|---|
| TJR101226 | 37.69 | 2.73 | 34.79 | 0.70 | 41.94 | 1.48 |
| TJR101227 | 34.02 | 0.07 | 42.27 | 1.16 | 72.19 | 0.23 |
| TJR101228 | 67.07 | 4.83 | 82.60 | 1.75 | 84.23 | 10.55 |
| TJR101229 | 47.09 | 0.33 | 65.75 | 0.77 | 86.99 | 5.06 |
| TJR101230 | 38.86 | 0.15 | 47.63 | 4.44 | 84.38 | 0.39 |
| TJR101231 | 30.63 | 8.47 | 40.31 | 0.26 | 69.28 | 2.09 |
| TJR101232 | 21.38 | 0.60 | 38.19 | 3.84 | 64.82 | 1.48 |
| TJR101233 | 76.38 | 6.11 | 55.63 | 0.52 | 74.82 | 3.58 |
| TJR101234 | 33.13 | 5.11 | 67.54 | 1.67 | 84.07 | 3.04 |
| TJR101236 | 49.92 | 4.38 | 69.74 | 2.47 | 86.49 | 5.33 |
| TJR101237 | 42.98 | 4.04 | 66.94 | 5.24 | 96.84 | 3.52 |
| TJR101239 | 44.77 | 3.85 | 65.84 | 16.68 | 89.33 | 2.32 |
| TJR101240 | 38.09 | 2.16 | 37.09 | 3.69 | 57.44 | 2.19 |
| TJR101242 | 41.42 | 0.57 | 41.34 | 4.69 | 63.08 | 2.46 |
| TJR101243 | 22.24 | 1.91 | 45.89 | 6.42 | 74.62 | 0.39 |
| TJR101244 | 72.55 | 0.13 | 64.40 | 4.05 | 68.89 | 1.97 |
| TJR101245 | 43.00 | 9.76 | 67.73 | 4.25 | 91.02 | 10.46 |
| TJR101248 | 28.13 | 0.78 | 31.8 | 2.75 | 58.28 | 1.17 |
| TJR101249 | 16.04 | 3.16 | 22.60 | 1.62 | 52.09 | 1.25 |
| TJR101250 | 36.42 | 1.48 | 39.90 | 1.81 | 72.92 | 3.06 |
| TJR101251 | 38.43 | 5.44 | 54.82 | 0.09 | 81.95 | 3.91 |
| TJR101252 | 57.45 | 1.31 | 70.59 | 1.34 | 88.85 | 6.10 |
| TJR101253 | 49.99 | 3.53 | 44.83 | 1.20 | 79.05 | 1.48 |
| TJR101255 | 70.28 | 6.88 | 55.51 | 1.50 | 72.12 | 4.37 |
| TJR101256 | 82.12 | 3.88 | 98.34 | 8.81 | 104.34 | 10.68 |
| TJR101257 | 36.95 | 0.96 | 64.55 | 2.16 | 91.83 | 5.01 |
| TJR101258 | 35.73 | 1.58 | 37.33 | 1.50 | 52.90 | 0.94 |
| TJR101259 | 57.16 | 3.61 | 56.99 | 1.38 | 57.90 | 7.03 |
| TJR101260 | 37.37 | 0.89 | 56.24 | 0.45 | 93.29 | 5.59 |
| TJR101261 | 32.42 | 7.50 | 31.80 | 1.65 | 59.23 | 3.20 |
| TJR101262 | 30.21 | 1.87 | 35.86 | 1.46 | 64.50 | 0.40 |
| TJR101263 | 47.34 | 6.64 | 69.22 | 0.25 | 106.57 | 1.85 |
| TJR101264 | 34.23 | 1.86 | 46.83 | 6.94 | 86.70 | 4.45 |
| TJR101266 | 57.35 | 1.14 | 65.30 | 0.34 | 98.35 | 1.35 |
| TJR101267 | 37.14 | 0.73 | 44.80 | 1.42 | 84.78 | 2.03 |

### Example 4. Inhibition of Human MAPT mRNA in MCF7 Cells by dsRNAs of Present Disclosure

The dsRNAs of the present disclosure were subjected to *in vitro* molecular-level activity screening in MCF7 cells. The initial final concentration for transfection of each dsRNA sample was 10 nM, and a 5-fold serial dilution was performed to obtain 7-9 concentration points.

MCF7 cells were cultured at 37 °C with 5% CO₂ in a Dulbecco's modified eagle medium containing 10% fetal bovine serum. 24 h prior to transfection, the MCF7 cells were seeded into a 96-well plate at a density of 1 × 10⁴ cells per well, with each well containing 100 µL of the culture medium.

The cells were transfected with the dsRNAs using Lipofectamine RNAi MAX (ThermoFisher, 13778150) according to the product instructions. A total of 7 or 9 concentration points were set for the dsRNAs, the initial concentration was 10 nM, and two duplicate wells were set for each concentration. 48 h after treatment, total cell RNA extraction was performed using an FG0417-L/FG0418-XL high-throughput cell RNA extraction kit (FireGen, magnetic bead method), and RNA reverse transcription (Takara, RR037A) and quantitative real-time PCR (Thermo, 4444557) detection were performed to determine the mRNA level of human MAPT. The mRNA level of human MAPT was corrected based on the GAPDH internal reference gene level.

For the real-time quantitative PCR detection, a probe Q-PCR detection experiment was used. Information on the primers is shown in Table 8 above.

**Table 8. Taqman primers**

| Primer name | SEQ ID NO: | Primer sequence (5'-3') |
|---|---|---|
| hMAPT-Forward | 295 | GACAGAGTCCAGTCGAAGATTG |
| hMAPT-Reverse | 296 | GCGACTTGTACACGATCTCC |
| hMAPT-Probe | 297 | (6-FAM)-     CGAGAACGCCAAAGCCAAGACAGA- (BHQ1) |
| hGAPDH-Forward | 298 | GACCCCTTCATTGACCTCAACTAC |
| hGAPDH-Reverse | 299 | TTGACGGTGCCATGGAATTT |
| hGAPDH-Probe | 300 | (VIC)-TTACATGTTCCAATATGATTCC-(BHQ1) |

### Results analysis method:

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%.

The results are expressed relative to the remaining percentage of human MAPT mRNA expression in cells treated with the dsRNAs. The inhibition rate IC₅₀ results are shown in Tables 9 and 10. The results in Tables 9 and 10 show that the dsRNAs of the present disclosure all exhibited good inhibitory activity against MAPT.

**Table 9. The activity of dsRNAs at 9 concentration points in MCF-7 cells (IC₅₀)**

| Remaining percentage of MAPT mRNA expression (mean) | | | | | |
|---|---|---|---|---|---|
| Double strand code | 10.00000 nM | 2.00000 nM | 0.40000 nM | 0.08000 nM | 0.01600 nM |
| TJR101212 | 9.0% | 12.8% | 16.3% | 28.4% | 58.5% |
| TJR101225 | 21.4% | 22.9% | 24.4% | 36.4% | 60.3% |
| TJR101226 | 21.4% | 22.9% | 24.4% | 36.4% | 60.3% |
| TJR101227 | 21.6% | 22.3% | 37.3% | 60.8% | 80.1% |
| TJR101230 | 16.7% | 24.0% | 39.9% | 63.3% | 83.5% |
| TJR101231 | 14.5% | 18.2% | 29.3% | 54.0% | 86.7% |
| TJR101232 | 11.2% | 13.0% | 24.2% | 51.1% | 81.7% |
| TJR101235 | 9.0% | 18.3% | 41.4% | 68.9% | 93.0% |
| TJR101242 | 19.6% | 17.5% | 26.2% | 42.9% | 72.9% |
| TJR101243 | 16.5% | 20.4% | 37.9% | 67.1% | 86.6% |
| TJR101248 | 24.1% | 20.5% | 24.6% | 50.6% | 78.6% |
| TJR101258 | 4.9% | 10.5% | 19.6% | 38.2% | 66.0% |
| TJR101261 | 16.8% | 15.3% | 19.6% | 34.3% | 61.8% |
| TJR101262 | 21.0% | 20.5% | 24.5% | 49.0% | 81.6% |
| TJR101264 | 6.3% | 10.6% | 27.2% | 62.0% | 89.2% |
| Double strand code | 0.00320 nM | 0.00064 nM | 0.00013 nM | 0.00003 nM | IC50 (nM) |
| TJR101212 | 87.1% | 94.1% | 96.4% | 95.7% | 0.025 |
| TJR101225 | 87.9% | 88.0% | 90.7% | 95.1% | 0.030 |
| TJR101226 | 87.9% | 88.0% | 90.7% | 95.1% | 0.020 |
| TJR101227 | 87.5% | 88.4% | 90.4% | 90.5% | 0.157 |
| TJR101230 | 90.8% | 87.4% | 86.6% | 93.8% | 0.202 |
| TJR101231 | 96.9% | 99.6% | 100.8% | 98.3% | 0.104 |
| TJR101232 | 90.1% | 97.4% | 98.1% | 95.4% | 0.086 |
| TJR101235 | 101.2% | 97.9% | 95.0% | 100.6% | 0.243 |
| TJR101242 | 89.8% | 97.1% | 96.6% | 87.0% | 0.055 |
| TJR101243 | 95.3% | 98.3% | 94.8% | 98.5% | 0.196 |
| TJR101248 | 96.5% | 97.3% | 99.5% | 97.8% | 0.074 |
| TJR101258 | 91.2% | 93.1% | 98.6% | 100.3% | 0.043 |
| TJR101261 | 85.4% | 90.8% | 88.8% | 94.6% | 0.031 |
| TJR101262 | 97.3% | 99.9% | 100.7% | 101.7% | 0.073 |
| TJR101264 | 97.6% | 93.5% | 103.7% | 98.2% | 0.265 |

**Table 10. The activity of dsRNAs at 7 concentration points in MCF-7 cells (IC₅₀)**

| Remaining percentage of MAPT mRNA expression (mean) | | | | |
|---|---|---|---|---|
| Double strand code | 10.00000 nM | 2.00000 nM | 0.40000 nM | 0.08000 nM |
| TJR101208 | 40.7% | 32.6% | 43.2% | 69.6% |
| TJR101209 | 19.5% | 28.6% | 55.0% | 77.2% |
| TJR101210 | 9.4% | 22.0% | 45.1% | 68.0% |
| TJR101211 | 23.0% | 28.9% | 53.0% | 79.3% |
| TJR101213 | 11.5% | 22.4% | 49.3% | 73.8% |
| TJR101214 | 15.4% | 24.3% | 42.8% | 71.0% |
| TJR101225 | 19.4% | 20.4% | 27.1% | 45.1% |
| TJR101233 | 26.2% | 28.9% | 44.4% | 75.1% |

| Double strand code | 0.01600 nM | 0.00320 nM | 0.00064 nM | IC₅₀ (nM) |
|---|---|---|---|---|
| TJR101208 | 96.8% | 99.9% | 111.1% | 0.224 |
| TJR101209 | 92.4% | 95.0% | 96.9% | 0.493 |
| TJR101210 | 86.9% | 95.5% | 97.6% | 0.280 |
| TJR101211 | 91.3% | 93.8% | 93.2% | 0.454 |
| TJR101213 | 92.1% | 93.8% | 98.2% | 0.363 |
| TJR101214 | 102.1% | 110.3% | 110.9% | 0.252 |
| TJR101225 | 83.3% | 91.7% | 96.8% | 0.068 |
| TJR101233 | 86.4% | 92.3% | 88.1% | 0.296 |

### Example 5. Inhibitory Activity of dsRNAs of Present Disclosure Against Human MAPT in A172 Cells

The dsRNAs of the present disclosure were subjected to *in vitro* molecular-level activity screening in A172 cells. The initial final concentration for transfection of each dsRNA sample was 10 nM, and a 5-fold serial dilution was performed to obtain 7-9 concentration points.

A172 cells were cultured at 37 °C with 5% CO₂ in a Dulbecco's modified eagle medium containing 10% fetal bovine serum. 24 h prior to transfection, the A172 cells were seeded into a 96-well plate at a density of 1 × 10⁴ cells per well, with each well containing 100 µL of the culture medium.

Refer to Example 4 for the experimental procedure and analysis of results. For the real-time quantitative PCR detection, a probe Q-PCR detection experiment was used. Information on the primers is shown in Table 8.

The dsRNA sequences were subjected to *in vitro* molecular-level activity screening in A172 cells using 7-9 concentration gradients. The results are expressed relative to the remaining percentage of human MAPT mRNA expression in cells treated with the control dsRNA. The inhibition rate IC₅₀ results are shown in Tables 11 and 12.

**Table 11. The activity of the dsRNAs of the present disclosure at 9 concentration points in A172 cells (IC₅₀)**

| Remaining percentage of MAPT mRNA expression (mean) | | | | | |
|---|---|---|---|---|---|
| Double strand code | 10.00000 nM | 2.00000 nM | 0.40000 nM | 0.08000 nM | 0.01600 nM |
| TJR101212 | 12.3% | 10.5% | 12.2% | 21.6% | 45.5% |
| TJR101225 | 15.8% | 14.3% | 13.0% | 19.6% | 43.2% |
| TJR101226 | 13.5% | 13.0% | 12.7% | 18.3% | 36.6% |
| TJR101227 | 19.7% | 16.9% | 27.0% | 58.5% | 85.9% |
| TJR101230 | 37.1% | 21.0% | 31.7% | 49.9% | 83.1% |
| TJR101231 | 12.9% | 11.1% | 17.5% | 38.7% | 77.1% |
| TJR101232 | 9.1% | 6.5% | 9.7% | 30.4% | 68.2% |
| TJR101235 | 13.9% | 11.1% | 13.0% | 29.4% | 70.4% |
| TJR101242 | 28.6% | 24.5% | 21.0% | 32.1% | 64.0% |
| TJR101243 | 23.6% | 21.9% | 26.5% | 54.9% | 86.0% |
| TJR101248 | 23.7% | 21.3% | 19.3% | 28.2% | 50.9% |
| TJR101258 | 2.2% | 3.3% | 8.8% | 23.4% | 48.5% |
| TJR101261 | 22.5% | 18.5% | 21.6% | 40.9% | 76.7% |
| TJR101262 | 23.7% | 19.9% | 19.8% | 26.4% | 61.0% |
| TJR101264 | 15.9% | 11.5% | 13.6% | 35.9% | 76.2% |

| Double strand code | 0.00320 nM | 0.00064 nM | 0.00013 nM | 0.00003 nM | IC50 (nM) |
|---|---|---|---|---|---|
| TJR101212 | 81.6% | 102.5% | 106.4% | 97.9% | 0.013 |
| TJR101225 | 76.3% | 87.7% | 86.3% | 93.5% | 0.012 |
| TJR101226 | 74.8% | 93.8% | 100.6% | 100.2% | 0.009 |
| TJR101227 | 95.1% | 97.0% | 103.2% | 106.2% | 0.109 |
| TJR101230 | 89.1% | 92.0% | 96.2% | 97.0% | 0.079 |
| TJR101231 | 96.6% | 104.7% | 100.7% | 107.3% | 0.049 |
| TJR101232 | 90.0% | 100.2% | 101.4% | 98.6% | 0.033 |
| TJR101235 | 84.0% | 89.3% | 101.1% | 97.2% | 0.033 |
| TJR101242 | 85.4% | 94.3% | 102.1% | 95.3% | 0.027 |
| TJR101243 | 92.5% | 93.0% | 89.5% | 102.5% | 0.095 |
| TJR101248 | 89.9% | 106.9% | 106.1% | 104.6% | 0.017 |
| TJR101258 | 76.2% | 85.8% | 94.3% | 102.1% | 0.015 |
| TJR101261 | 94.2% | 95.8% | 93.4% | 105.8% | 0.033 |
| TJR101262 | 92.1% | 104.0% | 103.7% | 102.0% | 0.023 |
| TJR101264 | 97.7% | 95.2% | 97.9% | 97.0% | 0.045 |

**Table 12. The activity of the dsRNAs of the present disclosure at 7 concentration points in A172 cells (IC₅₀)**

| Remaining percentage of MAPT mRNA expression (mean) | | | | |
|---|---|---|---|---|
| Double strand code | 10.00000 nM | 2.00000 nM | 0.40000 nM | 0.08000 nM |
| TJR101203 | 13.6% | 12.3% | 14.9% | 22.8% |
| TJR101205 | 13.5% | 15.5% | 30.8% | 67.2% |
| TJR101208 | 33.4% | 31.2% | 28.4% | 43.7% |
| TJR101209 | 18.9% | 19.3% | 21.9% | 49.5% |
| TJR101210 | 13.7% | 11.8% | 22.7% | 57.5% |
| TJR101211 | 40.4% | 42.4% | 36.8% | 58.3% |
| TJR101213 | 24.8% | 19.6% | 25.4% | 51.2% |
| TJR101214 | 17.7% | 19.1% | 17.2% | 29.0% |
| TJR101219 | 23.2% | 25.6% | 34.6% | 57.9% |
| TJR101229 | 27.7% | 26.3% | 48.5% | 91.6% |
| TJR101233 | 20.7% | 21.3% | 23.2% | 36.9% |
| TJR101234 | 14.4% | 13.7% | 28.3% | 67.0% |
| TJR101228 | 29.3% | 29.5% | 45.3% | 66.0% |
| TJR101236 | 25.8% | 18.8% | 24.3% | 35.6% |
| TJR101257 | 23.8% | 21.8% | 27.9% | 51.9% |
| TJR101263 | 14.5% | 15.0% | 24.0% | 50.4% |

| Double strand code | 0.01600 nM | 0.00320 nM | 0.00064 nM | IC50 (nM) |
|---|---|---|---|---|
| TJR101203 | 63.5% | 91.7% | 100.3% | 0.024 |
| TJR101205 | 91.3% | 97.8% | 109.1% | 0.158 |
| TJR101208 | 82.7% | 96.6% | 107.2% | 0.057 |
| TJR101209 | 86.2% | 104.2% | 103.7% | 0.075 |
| TJR101210 | 98.4% | 106.5% | 126.8% | 0.103 |
| TJR101211 | 94.6% | 90.0% | 108.5% | 0.115 |
| TJR101213 | 81.6% | 84.0% | 103.5% | 0.079 |
| TJR101214 | 65.0% | 85.2% | 95.2% | 0.028 |
| TJR101219 | 87.8% | 97.5% | 94.4% | 0.124 |
| TJR101229 | 97.1% | 112.3% | 110.5% | 0.380 |
| TJR101233 | 70.6% | 93.8% | 106.6% | 0.039 |
| TJR101234 | 84.8% | 96.4% | 101.9% | 0.146 |
| TJR101228 | 86.5% | 93.9% | 98.6% | 0.246 |
| TJR101236 | 61.4% | 79.6% | 87.7% | 0.030 |
| TJR101257 | 85.8% | 94.4% | 96.7% | 0.087 |
| TJR101263 | 86.0% | 96.1% | 101.3% | 0.083 |

### Example 6. psiCHECK On-Target Activity of dsRNAs

The dsRNAs of the present disclosure were subjected to an *in vitro* molecular-level simulation of on-target activity screening in HEK293A cells using 9 concentration gradients.

On-target GSCM plasmids of the dsRNA target sequence were constructed and inserted into psiCHECK-2 plasmids. The plasmids contained the renilla luciferase gene and the firefly luciferase gene. In the dual reporter gene system, the target sequence of the dsRNAs was inserted into the 3' UTR region of the renilla luciferase gene. The activity of the dsRNAs for the target sequence can be reflected by measurement of renilla luciferase expression corrected with firefly luciferase.

HEK293A cells were cultured at 37 °C with 5% CO₂ in a DMEM high-glucose culture medium containing 10% fetal bovine serum. 24 h prior to transfection, the HEK293A cells were seeded into a 96-well plate at a density of 8 × 10³ cells per well, with each well containing 100 µL of the culture medium.

The cells were co-transfected with the dsRNAs and corresponding GSCM plasmids using Lipofectamine2000 (ThermoFisher, 11668019) according to the instructions, with 0.2 µL of Lipofectamine2000 used per well. The amount of plasmids for transfection was 20 ng per well. For the on-target sequence plasmids, a total of 9 concentration points were set for the dsRNAs. The initial concentration was 20 nM, and a 4-fold serial dilution was performed. Two duplicate wells were set for each concentration. 24 h after transfection, the on-target levels were determined using a Dual-Luciferase Reporter Assay System (Promega, E2940).

**Table 13. The activity of the dsRNAs of the present disclosure at 9 concentration points in a psi-Check system (IC₅₀)**

| Double strand No. | Remaining percentage of MAPT mRNA (GSCM) expression (mean) | | | | |
|---|---|---|---|---|---|
| | 20 nM | 5 nM | 1.25 nM | 0.3125 nM | 0.07813 nM |
| TJR101212 | 30.4% | 17.9% | 16.7% | 23.9% | 45.5% |
| TJR101225 | 19.0% | 19.2% | 19.0% | 26.2% | 46.1% |
| TJR101226 | 11.8% | 14.8% | 16.7% | 20.1% | 44.0% |
| TJR101227 | 17.3% | 18.2% | 28.7% | 45.3% | 77.2% |
| TJR101230 | 70.5% | 38.7% | 23.3% | 28.5% | 50.0% |
| TJR101231 | 12.1% | 10.7% | 11.7% | 15.7% | 40.7% |
| TJR101232 | 7.8% | 9.0% | 11.8% | 20.6% | 41.4% |
| TJR101235 | 35.0% | 27.4% | 24.8% | 32.0% | 60.4% |
| TJR101242 | 16.4% | 19.4% | 22.0% | 31.1% | 50.6% |
| TJR101243 | 24.8% | 29.6% | 34.7% | 57.5% | 77.6% |
| TJR101248 | 7.7% | 8.0% | 10.4% | 16.3% | 31.2% |
| TJR101258 | 26.9% | 22.6% | 20.1% | 27.2% | 51.6% |
| TJR101261 | 11.7% | 13.7% | 14.1% | 21.8% | 43.2% |
| TJR101262 | 13.8% | 12.9% | 16.8% | 23.5% | 50.0% |
| TJR101264 | 29.4% | 17.9% | 18.6% | 23.9% | 50.0% |

| Double strand No. | Remaining percentage of MAPT mRNA (GSCM) expression (mean) | | | | |
|---|---|---|---|---|---|
| | 0.01953 nM | 0.00488 nM | 0.00122 nM | 0.00031 nM | IC₅₀ (nM) |
| TJR101212 | 74.8% | 92.8% | 99.8% | 97.0% | 0.058 |
| TJR101225 | 74.1% | 95.6% | 97.3% | 99.5% | 0.063 |
| TJR101226 | 75.4% | 88.1% | 91.9% | 97.9% | 0.059 |
| TJR101227 | 85.8% | 94.0% | 100.3% | 88.6% | 0.276 |
| TJR101230 | 81.8% | 96.1% | 101.8% | 102.4% | 0.072 |
| TJR101231 | 74.7% | 103.1% | 97.3% | 104.4% | 0.052 |
| TJR101232 | 81.2% | 95.5% | 103.7% | 98.9% | 0.061 |
| TJR101235 | 91.4% | 101.5% | 100.8% | 106.2% | 0.110 |
| TJR101242 | 76.8% | 84.3% | 89.6% | 93.5% | 0.086 |
| TJR101243 | 85.0% | 96.4% | 103.4% | 97.5% | 0.462 |
| TJR101248 | 56.7% | 88.0% | 94.9% | 96.0% | 0.030 |
| TJR101258 | 74.0% | 84.4% | 89.2% | 95.5% | 0.074 |
| TJR101261 | 69.2% | 86.3% | 100.3% | 103.7% | 0.050 |
| TJR101262 | 85.1% | 97.9% | 98.4% | 95.6% | 0.080 |
| TJR101264 | 81.1% | 96.4% | 98.4% | 94.6% | 0.073 |

**Table 14. The activity of the dsRNAs of the present disclosure at 9 concentration points in a psi-Check system (IC₅₀)**

| Double strand No. | Remaining percentage of MAPT mRNA (GSCM) expression (mean) | | | | |
|---|---|---|---|---|---|
| | 20 nM | 5 nM | 1.25 nM | 0.3125 nM | 0.07813 nM |
| TJR101208 | 19.0% | 20.5% | 24.6% | 34.2% | 64.5% |
| TJR101209 | 11.8% | 17.2% | 21.3% | 27.7% | 50.6% |
| TJR101219 | 36.7% | 37.4% | 40.5% | 52.9% | 72.2% |
| TJR101224 | 51.5% | 43.9% | 42.8% | 57.0% | 83.1% |
| TJR101228 | 32.5% | 31.6% | 45.0% | 61.1% | 89.3% |
| TJR101229 | 76.3% | 46.5% | 30.7% | 33.4% | 55.3% |
| TJR101233 | 29.5% | 32.1% | 30.9% | 33.2% | 56.0% |
| TJR101234 | 32.9% | 30.1% | 28.6% | 36.2% | 60.6% |
| TJR101236 | 56.7% | 46.5% | 42.6% | 46.4% | 70.3% |
| TJR101257 | 28.4% | 28.1% | 36.0% | 54.6% | 82.5% |
| TJR101263 | 40.1% | 39.9% | 45.0% | 60.9% | 86.2% |
| TJR101203 | 35.1% | 34.8% | 42.6% | 50.5% | 64.9% |
| TJR101210 | 45.9% | 26.8% | 18.6% | 31.4% | 52.6% |
| TJR101211 | 23.0% | 25.3% | 27.9% | 37.5% | 57.5% |
| TJR101213 | 19.2% | 15.8% | 18.8% | 26.8% | 49.9% |
| TJR101214 | 26.5% | 23.4% | 28.2% | 45.0% | 68.3% |

| 0.01953 nM | 0.00488 nM | 0.00122 nM | 0.00031 nM | IC₅₀ (nM) | |
|---|---|---|---|---|---|
| TJR101208 | 91.1% | 108.0% | 106.2% | 115.0% | 0.140 |
| TJR101209 | 79.3% | 88.2% | 91.6% | 92.7% | 0.084 |
| TJR101219 | 100.6% | 104.8% | 100.3% | 105.6% | 0.315 |
| TJR101224 | 88.9% | 93.8% | 96.8% | 94.1% | 0.557 |
| TJR101228 | 97.3% | 94.9% | 98.2% | 98.6% | 0.653 |
| TJR101229 | 79.4% | 93.2% | 97.9% | 103.5% | 0.092 |
| TJR101233 | 77.7% | 98.0% | 103.2% | 101.0% | 0.094 |
| TJR101234 | 85.7% | 93.5% | 101.3% | 100.8% | 0.124 |
| TJR101236 | 94.2% | 98.9% | 105.1% | 99.6% | 0.304 |
| TJR101257 | 92.8% | 100.8% | 106.0% | 103.2% | 0.440 |
| TJR101263 | 97.2% | 104.4% | 104.7% | 94.1% | 0.656 |
| TJR101203 | 86.7% | 96.6% | 104.8% | 98.2% | 0.295 |
| TJR101210 | 82.7% | 102.5% | 92.2% | 98.8% | 0.087 |
| TJR101211 | 84.9% | 99.4% | 99.0% | 99.9% | 0.122 |
| TJR101213 | 82.8% | 98.4% | 94.1% | 101.7% | 0.079 |
| TJR101214 | 83.8% | 96.5% | 96.8% | 103.8% | 0.211 |

### Example 7. Inhibitory Activity of dsRNAs Against Human MAPT in A172 Cells

The dsRNAs of the present disclosure were subjected to *in vitro* molecular-level activity screening in A172 cells. The initial final concentration for transfection of each dsRNA sample was 10 nM, and a 5-fold serial dilution was performed to obtain 7 concentration points.

A172 cells were cultured at 37 °C with 5% CO₂ in a Dulbecco's modified eagle medium containing 10% fetal bovine serum. 24 h prior to transfection, the A172 cells were seeded into a 96-well plate at a density of 1 × 10⁴ cells per well, with each well containing 100 µL of the culture medium.

Refer to Example 4 for the experimental procedure and analysis of results. For the real-time quantitative PCR detection, a probe Q-PCR detection experiment was used. Information on the primers is shown in Table 6.

The dsRNA sequences were subjected to *in vitro* molecular-level activity screening in A172 cells using 7 concentration gradients. The results are expressed relative to the remaining percentage of human MAPT mRNA expression in cells treated with the control dsRNA. The inhibition rate IC₅₀ results are shown in Table 15. The results in Table 15 show that the TJR103374 of the present disclosure exhibited significantly superior inhibitory activity against MAPT mRNA compared to TJR103646, TJR103375, and TJR103376.

**Table 15-1. The activity of dsRNAs at 7 concentration points in A172 cells (IC50)**

| Remaining percentage of MAPT mRNA expression (mean) | | | | |
|---|---|---|---|---|
| Double strand code | 10.00000 nM | 2.00000 nM | 0.40000 nM | 0.08000 nM |
| TJR103374 | 7.9% | 4.9% | 6.3% | 7.9% |
| TJR103646 | 10.9% | 10.5% | 12.2% | 17.3% |

| Double strand code | 0.01600 nM | 0.00320 nM | 0.00064 nM | IC50 (nM) |
|---|---|---|---|---|
| TJR103374 | 16.9% | 46.3% | 75.3% | 0.0027 |
| TJR103646 | 37.2% | 69.1% | 88.2% | 0.0083 |

**Table 15-2. The activity of dsRNAs at 7 concentration points in A172 cells (IC50)**

| Remaining percentage of MAPT mRNA expression (mean) | | | | |
|---|---|---|---|---|
| Double strand code | 10.00000 nM | 2.00000 nM | 0.40000 nM | 0.08000 nM |
| TJR103375 | 36.7% | 36.2% | 40.2% | 66.5% |
| TJR103376 | 10.5% | 17.9% | 37.9% | 76.6% |

| Double strand code | 0.01600 nM | 0.00320 nM | 0.00064 nM | IC50 (nM) |
|---|---|---|---|---|
| TJR103375 | 95.7% | 107.9% | 116.5% | 0.197 |
| TJR103376 | 106.5% | 114.8% | 115.7% | 0.232 |

### Example 8. psiCHECK On-Target Activity of dsRNAs

The dsRNAs of the present disclosure were subjected to an *in vitro* molecular-level simulation of on-target activity screening in HEK293A cells using 9 concentration gradients.

On-target GSCM plasmids of the dsRNA target sequence were constructed and inserted into psiCHECK-2 plasmids. The plasmids contained the renilla luciferase gene and the firefly luciferase gene. In the dual reporter gene system, the target sequence of the dsRNAs was inserted into the 3' UTR region of the renilla luciferase gene. The activity of the dsRNAs for the target sequence can be reflected by measurement of renilla luciferase expression corrected with firefly luciferase.

HEK293A cells were cultured at 37 °C with 5% CO₂ in a DMEM high-glucose culture medium containing 10% fetal bovine serum. 24 h prior to transfection, the HEK293A cells were seeded into a 96-well plate at a density of 8 × 10³ cells per well, with each well containing 100 µL of the culture medium.

The cells were co-transfected with the dsRNAs and corresponding GSCM plasmids using Lipofectamine2000 (ThermoFisher, 11668019) according to the instructions, with 0.2 µL of Lipofectamine2000 used per well. The amount of plasmids for transfection was 20 ng per well. For the on-target sequence plasmids, a total of 9 concentration points were set for the dsRNAs. The initial concentration was 20 nM, and a 3-fold serial dilution was performed. Two duplicate wells were set for each concentration. 24 h after transfection, the on-target levels were determined using a Dual-Luciferase Reporter Assay System (Promega, E2940).

**Table 16. The activity of the dsRNAs of the present disclosure at 9 concentration points in a psi-Check system (IC₅₀)**

| Double strand No. | Remaining percentage of MAPT mRNA (GSCM) expression (mean) | | | | |
|---|---|---|---|---|---|
| | 20 nM | 6.67 nM | 2.22 nM | 0.74 nM | 0.25 nM |
| TJR103599 | 17.3% | 16.4% | 16.5% | 18.3% | 20.2% |
| TJR103600 | 19.5% | 23.9% | 26.3% | 26.1% | 26.4% |
| TJR102156 | 25.9% | 28.8% | 24.0% | 25.8% | 26.3% |
| TJR103601 | 17.7% | 16.2% | 13.9% | 20.4% | 16.0% |
| TJR103602 | 12.4% | 14.1% | 17.6% | 16.4% | 18.4% |
| TJR103603 | 23.8% | 24.9% | 24.6% | 29.3% | 28.4% |
| TJR103604 | 14.9% | 18.8% | 17.7% | 20.5% | 21.2% |
| TJR103605 | 16.5% | 18.4% | 21.5% | 22.2% | 20.2% |
| TJR102157 | 24.3% | 26.6% | 25.9% | 27.4% | 29.9% |

| Double strand No. | Remaining percentage of MAPT mRNA (GSCM) expression (mean) | | | | |
|---|---|---|---|---|---|
| | 0.082 nM | 0.027 nM | 0.0091 nM | 0.0031 nM | IC50 (nM) |
| TJR103599 | 24.9% | 38.6% | 57.3% | 86.0% | 0.013 |
| TJR103600 | 33.3% | 41.1% | 70.6% | 97.4% | 0.020 |
| TJR102156 | 31.9% | 42.0% | 55.4% | 84.3% | 0.013 |
| TJR103601 | 17.0% | 24.6% | 39.8% | 58.3% | 0.005 |
| TJR103602 | 20.4% | 31.1% | 51.8% | 73.2% | 0.010 |
| TJR103603 | 36.5% | 52.9% | 67.4% | 95.2% | 0.027 |
| TJR103604 | 33.3% | 55.3% | 81.2% | 81.8% | 0.037 |
| TJR103605 | 22.7% | 31.9% | 51.3% | 78.4% | 0.010 |
| TJR102157 | 38.9% | 56.0% | 75.9% | 96.5% | 0.037 |

### Example 9. Inhibitory Activity of dsRNAs Against Human MAPT in A172 Cells

The dsRNAs of the present disclosure were subjected to *in vitro* molecular-level activity screening in A172 cells. The initial final concentration for transfection of each dsRNA sample was 10 nM, and a 5-fold serial dilution was performed to obtain 7 concentration points.

A172 cells were cultured at 37 °C with 5% CO₂ in a Dulbecco's modified eagle medium containing 10% fetal bovine serum. 24 h prior to transfection, the A172 cells were seeded into a 96-well plate at a density of 1 × 10⁴ cells per well, with each well containing 100 µL of the culture medium.

Refer to Example 4 for the experimental procedure and analysis of results. For the real-time quantitative PCR detection, a probe Q-PCR detection experiment was used. Information on the primers is shown in Table 6.

The dsRNA sequences were subjected to *in vitro* molecular-level activity screening in A172 cells using 7 concentration gradients. The results are expressed relative to the remaining percentage of human MAPT mRNA expression in cells treated with the control dsRNA. The inhibition rate IC₅₀ results are shown in Table 17. The results in Table 17 show that the dsRNAs of the present disclosure exhibited significantly excellent inhibitory activity against MAPT mRNA.

**Table 17. The activity of dsRNAs at 7 concentration points in A172 cells (IC50)**

| Remaining percentage of MAPT mRNA expression (mean) | | | | |
|---|---|---|---|---|
| Double strand code | 10.00000 nM | 2.00000 nM | 0.40000 nM | 0.08000 nM |
| TJR102182 | 12.2% | 11.3% | 12.1% | 22.3% |
| TJR103670 | 10.3% | 9.9% | 11.2% | 18.6% |
| TJR103671 | 9.4% | 6.6% | 7.5% | 11.1% |
| TJR103672 | 8.7% | 7.6% | 8.9% | 15.1% |
| TJR103673 | 18.9% | 16.2% | 18.3% | 34.6% |
| TJR103674 | 31.2% | 26.7% | 23.9% | 38.1% |
| TJR103675 | 13.3% | 9.4% | 15.1% | 38.3% |
| TJR103676 | 13.7% | 12.4% | 16.1% | 28.6% |
| TJR103677 | 6.0% | 5.5% | 9.7% | 22.3% |
| TJR102242 | 12.1% | 17.2% | 14.0% | 20.5% |

| Double strand code | 0.01600 nM | 0.00320 nM | 0.00064 nM | IC50 (nM) |
|---|---|---|---|---|
| TJR102182 | 53.4% | 89.4% | 100.5% | 0.0183 |
| TJR103670 | 46.6% | 82.7% | 105.1% | 0.0132 |
| TJR103671 | 35.8% | 76.3% | 97.8% | 0.0089 |
| TJR103672 | 41.9% | 76.5% | 99.1% | 0.0105 |
| TJR103673 | 74.3% | 98.1% | 103.7% | 0.0393 |
| TJR103674 | 81.0% | 93.6% | 96.2% | 0.0466 |
| TJR103675 | 81.6% | 95.3% | 96.0% | 0.0534 |
| TJR103676 | 64.1% | 89.0% | 96.5% | 0.0283 |
| TJR103677 | 64.6% | 88.7% | 96.0% | 0.0264 |
| TJR102242 | 58.0% | 94.0% | 105.5% | 0.020 |

## Claims

1. A dsRNA, comprising a sense strand and an antisense strand that form a double-stranded region, wherein:
the sense strand comprises a sequence of at least 15 contiguous nucleotides differing from any one of the nucleotide sequences of SEQ ID NO: 1 to SEQ ID NO: 10 by no more than 3 nucleotides; and
the antisense strand comprises a sequence of at least 15 contiguous nucleotides differing from any one of the nucleotide sequences of SEQ ID NO: 11 to SEQ ID NO: 20 by no more than 3 nucleotides.

2. The dsRNA according to claim 1, wherein the sense strand comprises a sequence of at least 17 contiguous nucleotides differing from any one of the nucleotide sequences of SEQ ID NO: 1 to SEQ ID NO: 10 by no more than 3 nucleotides, and/or the antisense strand comprises a sequence of at least 17 contiguous nucleotides differing from any one of the nucleotide sequences of SEQ ID NO: 11 to SEQ ID NO: 20 by no more than 3 nucleotides;
preferably, the sense strand comprises a sequence of at least 19 contiguous nucleotides differing from any one of the nucleotide sequences of SEQ ID NO: 1 to SEQ ID NO: 10 by no more than 3 nucleotides, and/or the antisense strand comprises a sequence of at least 21 contiguous nucleotides differing from any one of the nucleotide sequences of SEQ ID NO: 11 to SEQ ID NO: 20 by no more than 3 nucleotides.

3. The dsRNA according to claim 1 or 2, comprising or selected from the group consisting of any one of the following groups:
group 1): a sense strand set forth in SEQ ID NO: 1 and an antisense strand set forth in SEQ ID NO: 11;
group 2): a sense strand set forth in SEQ ID NO: 2 and an antisense strand set forth in SEQ ID NO: 12;
group 3): a sense strand set forth in SEQ ID NO: 3 and an antisense strand set forth in SEQ ID NO: 13;
group 4): a sense strand set forth in SEQ ID NO: 4 and an antisense strand set forth in SEQ ID NO: 14;
group 5): a sense strand set forth in SEQ ID NO: 5 and an antisense strand set forth in SEQ ID NO: 15;
group 6): a sense strand set forth in SEQ ID NO: 6 and an antisense strand set forth in SEQ ID NO: 16;
group 7): a sense strand set forth in SEQ ID NO: 7 and an antisense strand set forth in SEQ ID NO: 17;
group 8): a sense strand set forth in SEQ ID NO: 8 and an antisense strand set forth in SEQ ID NO: 18;
group 9): a sense strand set forth in SEQ ID NO: 9 and an antisense strand set forth in SEQ ID NO: 19; and
group 10): a sense strand set forth in SEQ ID NO: 10 and an antisense strand set forth in SEQ ID NO: 20.

4. The dsRNA according to any one of claims 1-3, wherein at least one nucleotide in the sense strand and/or the antisense strand is a modified nucleotide;
preferably, all nucleotides in the sense strand and/or the antisense strand are modified nucleotides.

5. The dsRNA according to claim 4, wherein three contiguous nucleotides in the sense strand are 2'-fluoro-modified nucleotides;
and/or
in the direction from the 5' end to the 3' end, each of the nucleotides at positions 2, 6, 12, 14, and 16 of the antisense strand is independently a 2'-fluoro-modified nucleotide; preferably, in the direction from the 5' end to the 3' end, the nucleotides at positions 7, 8, and 9 of the sense strand are 2'-fluoro-modified nucleotides; each of the nucleotides at positions 2, 6, 12, 14, and 16 of the antisense strand is independently a 2'-fluoro-modified nucleotide, or
the nucleotide at position 2, 4, 6, 10, 12, 14, 16, or 18 of the antisense strand is independently a 2'-fluoro-modified nucleotide; optionally, the 5' end of the antisense strand comprises one 5'-vinylphosphodiester group;
the nucleotides at the remaining positions of the sense strand and the antisense strand are 2-methoxy-modified nucleotides.

6. The dsRNA according to any one of claims 1-5, wherein at least one phosphodiester group in the sense strand and/or the antisense strand is a phosphodiester group with a modification group, preferably a phosphorothioate diester group.

7. The dsRNA according to claim 6, wherein the phosphodiester group with a modification group is present in at least one position selected from the group consisting of the following positions:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand;
preferably,
the sense strand comprises 1, 2, 3, or 4 phosphodiester groups with a modification group, preferably phosphorothioate diester groups; and/or
the antisense strand comprises 1, 2, 3, or 4 phosphodiester groups with a modification group, preferably phosphorothioate diester groups.

8. The dsRNA according to any one of claims 4-7, wherein the sense strand comprises the nucleotide sequence set forth in any one of SEQ ID NO: 137 to SEQ ID NO: 146; and/or the antisense strand comprises the nucleotide sequence set forth in any one of SEQ ID NO: 202 to SEQ ID NO: 211.

9. The dsRNA according to any one of claims 1-8, wherein the sense strand of the dsRNA is linked to one or more lipophilic groups.

10. The dsRNA according to claim 9, wherein the sense strand comprises the sequence set forth in any one of SEQ ID NO: 267 to SEQ ID NO: 276, and the antisense strand comprises the sequence set forth in any one of SEQ ID NO: 277 to SEQ ID NO: 286.

11. A pharmaceutical composition, comprising the dsRNA according to any one of claims 1-10 and a pharmaceutically acceptable carrier.

12. A cell, comprising the dsRNA according to any one of claims 1-10.

13. A kit, comprising the dsRNA according to any one of claims 1-10 or the pharmaceutical composition according to claim 11.

14. A method for reducing microtubule-associated protein tau gene (MAPT) expression, comprising administering to a subject an effective amount or effective dose of the dsRNA according to any one of claims 1-10 or the pharmaceutical composition according to claim 11.

15. A method for treating and/or preventing a disease associated with MAPT gene expression in a subject, comprising administering to the subject an effective amount or effective dose of the dsRNA according to any one of claims 1-10 or the pharmaceutical composition according to claim 11, wherein preferably, the disease associated with MAPT gene expression is selected from the group consisting of tauopathy, Alzheimer's disease (AD), frontotemporal dementia and parkinsonism linked to chromosome 17 (FTDP-17), frontotemporal dementia (FTD), progressive supranuclear palsy (PSP), corticobasal degeneration (CBD), chronic traumatic encephalopathy (CTE), epilepsy, Dravet syndrome (DS), Pick's disease (PiD), argyrophilic grain disease (AGD), and globular glial tauopathy (GGT).

16. A method for treating and/or preventing a disease, comprising administering to a subject an effective amount or effective dose of the dsRNA according to any one of claims 1-10 or the pharmaceutical composition according to claim 11, wherein preferably, the disease is selected from the group consisting of tauopathy, Alzheimer's disease (AD), frontotemporal dementia and parkinsonism linked to chromosome 17 (FTDP-17), frontotemporal dementia (FTD), progressive supranuclear palsy (PSP), corticobasal degeneration (CBD), chronic traumatic encephalopathy (CTE), epilepsy, Dravet syndrome (DS), Pick's disease (PiD), argyrophilic grain disease (AGD), and globular glial tauopathy (GGT).

17. A method for delivering a dsRNA inhibiting MAPT expression and/or replication *in vivo,* comprising administering to a subject the dsRNA according to any one of claims 1-10 or the pharmaceutical composition according to claim 11.

18. A method for preparing a dsRNA or a pharmaceutical composition, comprising synthesizing the dsRNA according to any one of claims 1-10 or the pharmaceutical composition according to claim 11.
